# EUROPÄISCHE PATENTANMELDUNG

(11) **EP 4 079 835 A1**
(43) Veröffentlichungstag der Anmeldung: **26.10.2022**
(21) Anmeldenummer: 22164960.1
(22) Anmeldetag: 29.03.2022
(51) Int. Cl.: C12M 1/00, C12M 1/34

(54) **LABORGERÄT**

(30) Priorität: 09.04.2021 DE 102021108910
(71) Anmelder: Thermo Electron LED GmbH, 63505 Langenselbold (DE)
(72) Erfinder: Schuck, Rainer, 63796 Kahl am Main (DE); Schneider, Jürgen, 63579 Freigericht (DE); Schäfer, Simone, 63571 Gelnhausen (DE); Barnkoth, Tino, 63776 Mömbris (DE)
(74) Vertreter: Stellbrink & Partner Patentanwälte mbB

(57) **Zusammenfassung**

Die Erfindung betrifft ein Laborgerät (1), wobei das Laborgerät (1) ein äußeres Gehäuse (10) aufweist, das einen Gerät-Innenraum definiert, wobei das Laborgerät (1) ausgelegt ist, einen Betriebszustand einzunehmen, bei dem ein Druck in dem Gerät-Innenraum geringer ist als ein Umgebungsdruck in der Umgebung des Laborgerätes (1). Die Erfindung betrifft auch die Verwendung des Laborgerätes in einem Reinraum.

## Beschreibung

Die vorliegende Erfindung betrifft ein Laborgerät. Insbesondere betrifft die vorliegende Erfindung Labortischgeräte, welche unter anderem Inkubatoren, Zentrifugen und biologische Sicherheitsschränke umfassen.

Solche Laborgeräte werden üblicherweise in Laboren eingesetzt und verfügen über unterschiedliche Funktionalitäten. Bei einigen Laborgeräten ist beispielsweise auch eine Temperaturkontrolle von Vorteil (beispielsweise bei Inkubatoren oder bei Zentrifugen). Beispielsweise um eine solche Temperaturkontrolle zu erreichen können Laborgeräte einen Luftaustausch zwischen dem Laborgerät und der Umgebung (beispielsweise dem Labor) bereitstellen. Hierbei ist es möglich, dass beispielsweise Partikel aus dem Laborgerät in die Umgebung abgegeben werden. Eine Abgabe von solchen Partikeln (und insbesondere eine große Partikelmenge) an die Umgebung kann in manchen Situationen nachteilig sein - insbesondere kann es, beispielsweise beim Arbeiten in Reinräumen - gewünscht sein, dass lediglich eine beschränkte Menge an Partikeln pro Zeiteinheit von dem Laborgerät an die Umgebung abgegeben werden.

Aktuelle Laborgeräte können diesen Anforderungen oftmals nicht genügen und geben eine zu große Menge an Partikeln pro Zeiteinheit an die Umgebung ab, was in vielen Situationen nachteilig sein kann.

Ausführungsformen der vorliegenden Erfindung sind auf die Aufgabe gerichtet, die Nachteile und Unzulänglichkeiten des Standes der Technik zu überwinden bzw. zumindest abzuschwächen. Dementsprechend ist es eine Aufgabe der vorliegenden Erfindung, ein Laborgerät bereitzustellen, dass relativ wenig Partikel an die Umgebung abgibt bzw. bei dem die Abgabe relativ kontrolliert stattfindet.

Diese Aufgaben werden durch das Laborgerät der vorliegenden Erfindung gelöst.

Gemäß einem Aspekt betrifft die vorliegende Erfindung ein Laborgerät, welches ein äußeres Gehäuse aufweist, das einen Gerät-Innenraum definiert. Das Laborgerät ist ausgelegt, einen Betriebszustand einzunehmen, bei dem ein Druck in dem Gerät-Innenraum geringer ist als ein Umgebungsdruck in der Umgebung des Laborgerätes.

Insbesondere kann das Laborgerät ausgebildet sein, einen vorbestimmten Differenzdruck in Bezug auf eine das Laborgerät umgebende Atmosphäre zumindest in einem Teil des Gerät-Innenraums zu erzeugen. Das äußere Gehäuse realisiert den Vorteil, dass Partikel, welche in dem Gerät-Innenraum erzeugt werden und/oder von den Materialien innerhalb des Laborgeräts in die Luft abgegeben werden, nicht unkontrolliert in die Laboratmosphäre abgegeben werden, sondern zunächst innerhalb des Laborgeräts gehalten werden. Mit einem geringeren Druck innerhalb des äußeren Gehäuses kann ein unkontrolliertes Ausströmen von Luft aus dem äußeren Gehäuse reduziert werden, sodass auch die in der Luft enthaltenen Partikel mehrheitlich oder vollständig innerhalb des äußeren Gehäuses verbleiben. Die mit Partikeln versetzte Luft innerhalb des Gerät-Innenraums kann mittels einer Filtervorrichtung von Partikeln befreit oder zumindest die Anzahl an Partikeln reduziert werden.

Partikel im Sinne der Erfindung umfassen kleine begrenzte Objekte, welche insbesondere von Feststoffen gelöst werden können und von einem gasförmigen Fluid, insbesondere Luft, getragen werden können. Partikel können ferner Aerosole, Staub, Feinstaub, flüchtige organische Verbindungen (VOC- volatile organic compounds), Nanopartikel und/oder Ultrafeinpartikel umfassen.

Vorteilhafterweise erreicht das Laborgerät eine reduzierte Partikelemission, sodass ein Betrieb des Laborgeräts innerhalb eines Reinraums ohne weitere Filtermaßnahmen möglich ist. Insbesondere kann das Laborgerät in einem Reinraum der ISO-Klasse 5 oder besser betrieben werden.

Damit eignet sich das entsprechende Laborgerät insbesondere auch für den Einsatz in einem Reinraum.

Diesbezüglich dürfte verständlich sein, dass es für Laborgeräte für den Betreib in einem reinen Bereich vorteilhaft ist, wenn die Kontamination der Luft, von Geräteoberflächen und/oder Laboroberflächen kontrolliert werden kann. Beispielsweise gibt es diesbezüglich ISO-Normen, aber auch nationale oder internationalen Richtlinien, beispielsweise die EU Richtline GMP Annex 1.

Ausführungsformen der vorliegenden Erfindung erlauben es insbesondere, Prozess- und Produktqualität reproduzierbar darzustellen, indem das Laborgerät den Einsatz in Reinräumen unter kontrollierten Bedingungen erlaubt, um so beispielsweise das Risiko einer Kontamination zu verringern.

Ausführungsformen der Erfindung erlauben es, die Partikelemission, respektive die Anzahl von Partikeln in dem Raum, in dem das Gerät verwendet wird, möglichst konstant bzw. gering zu halten, was insbesondere für die Verwendung des Gerätes in einem technischen Reinraum vorteilhaft ist. Je nach Anwendungsgebiet können Grenzwerte bezüglich der Partikelemission eines Geräts in Bezug auf die Partikelanzahl und/oder die Partikelgröße definiert werden. Für technische Reinräume kann insbesondere auf die Richtlinie ISO Norm 14644 verwiesen werden.

Entsprechend sollten in entsprechende Reinräume eingebrachte Geräte definierte Partikelemissionen aufweisen, welche die definierten Partikelemissionsgrenzwerte nicht überschreiten. Diese Funktionalität kann von Ausführungsformen der vorliegenden Erfindung bereitgestellt werden.

Laborgeräte können in Abhängigkeit von ihrer Funktion einen gewissen Luftaustausch mit der Laboratmosphäre aufweisen. Insbesondere Laborgeräte, welche beispielsweise eine Temperatursteuerung, Druckregulierungsfunktion und/oder Verdampfungsfunktion aufweisen, können einen Austausch mit der Laboratmosphäre aufweisen, sodass Partikel von dem Gerät in die Laboratmosphäre gelangen können. Der Luftaustausch kann in dem Laborgerät beispielsweise für eine Kühlung, Heizung, zum Evakuieren und/oder zum Belüften erforderlich sein. Dementsprechend kann die Erfindung insbesondere bei solchen Geräten Anwendung finden.

Je größer eine Temperaturdifferenz zwischen dem Laborgerät und der Umgebungstemperatur, desto höher kann eine Partikelemission des Laborgeräts sein. Ferner können Laborgeräte mit beweglichen Teilen in Abhängigkeit von einer Geschwindigkeit von Teilen des Laborgeräts eine erhöhte Partikelemission aufweisen. Entsprechend ist es vorteilhaft, insbesondere für Öfen, Sterilisatoren, Inkubatoren, Mischer und Zentrifugen, die Partikelemission zu bestimmen und entsprechend der Reinraumanforderungen zu reduzieren.

In Ausführungsformen der Erfindung ist es möglich, dass die Oberflächen in Kontakt mit der Laboratmosphäre möglichst emissionsarm in Bezug auf Partikel und kompatibel zu standardisierten Reinraumreinigungsverfahren sind.

Ferner können in Ausführungsformen der Erfindung bei einem Luftaustausch zwischen einem Luftvolumen in dem Laborgerät und der Laboratmosphäre der Partikeleintrag von der Laboratmosphäre in das Luftvolumen in dem Laborgerät reduziert werden. Das kann beispielsweise die Gefahr einer Probenkontamination innerhalb des Laborgeräts reduzieren. Insbesondere zielen Ausführungsformen der Erfindung darauf ab, dass die Atmosphäre in der Umgebung des Laborgerätes möglichst rein gehalten wird, indem die Partikelabgabe des Gerätes kontrolliert wird. Wird das Gerät in einer solchen Umgebung geöffnet (beispielsweise um Proben in das Gerät einzubringen), so ist das auch für die Reinheit innerhalb des Gerätes vorteilhaft, da die reine Luft in der Umgebung (zu der das Gerät selbst beiträgt) die Gefahr einer Kontamination des Innenraums absenkt.

Luftgetragene Partikel können schädlich für Laborproben, beispielsweise Zellen, und gefährlich für Laborpersonal sein. Mit Partikeln kontaminierte Proben oder Produkte können fehlerhaft sein, wobei ein Kontaminationsrisiko mit der Partikelkonzentration steigen kann. Insbesondere bei thermisch betriebenen Geräten können durch Luftströmungen unkontrolliert Partikel in die Laboratmosphäre abgegeben werden. Derartige Nachteile können mit Ausführungsformen der Erfindung vermieden werden.

Insgesamt dürfte es verständlich sein, dass Ausführungsformen der vorliegenden Erfindung die Reduktion von Partikelemissionen betrifft, beispielsweise in den Rahmen festgelegter Bereiche.

Insgesamt können mittels Ausführungsformen der Erfindung also verbesserte Laborgeräte bereitgestellt werden, die insbesondere auch für den Einsatz in Reinräumen geeignet sein können.

Das Laborgerät kann eine Differenz zwischen dem Umgebungsdruck und dem Druck in dem Gerät-Innenraum erzeugen, wobei die Druckdifferenz in einem Bereich von 1 Pa bis 1000 Pa, bevorzugt im Bereich von 2 Pa bis 500 Pa, weiter bevorzugt im Bereich von 5 Pa bis 400 Pa liegt.

Das Laborgerät kann eine Vorrichtung zur Erzeugung der Druckdifferenz aufweisen. Die Vorrichtung zur Erzeugung einer Druckdifferenz kann ausgebildet sein kontinuierlich eine Druckdifferenz zu erhalten. Dies kann den Vorteil erreichen, dass unabhängig von einem absoluten Atmosphärendruck der Umgebungsatmosphäre des Laborgeräts, in dem Laborgerät stets eine relative, insbesondere konstante Druckdifferenz erzeugt werden kann. Vorzugsweise erzeugt die Vorrichtung zur Erzeugung der Druckdifferenz einen Unterdruck, sodass durch nichtgedichtete Bereiche des Laborgeräts Luft in das Laborgerät eindringen kann, jedoch der Austritt von Luft aus nichtgedichteten Bereichen des Laborgeräts unterbunden oder zumindest reduziert werden kann. Insbesondere kann ein Austritt von Luft aus dem Laborgerät auf einen Auslass der Vorrichtung zur Erzeugung der Druckdifferenz konzentriert und/oder beschränkt werden.

Die Vorrichtung zur Erzeugung der Druckdifferenz kann ein Gebläse und/oder eine Pumpe umfassen. Das Gebläse erreicht den Vorteil, dass eine Luftströmung erzeugt werden kann, welche Luft innerhalb des Gerät-Innenraums in Richtung des Gebläses transportiert und dann aus dem Innenraum transportiert.

Die Vorrichtung zur Erzeugung der Druckdifferenz kann ausgelegt sein, eine Förderleistung im Bereich vom 10-fachen bis 40-fachen eines Gesamtvolumens des Laborgerätes pro Stunde zu erzielen, vorzugsweise im Bereich vom 15-fachen bis 30-fachen des Gesamtvolumens des Laborgerätes pro Stunde zu erzielen.. In Abhängigkeit von der Förderleistung kann eine Menge an Partikeln aus dem Gerät-Innenraum zu der Vorrichtung zur Erzeugung der Druckdifferenz transportiert werden. Mit einer Reduktion der Förderleistung kann entsprechend die Menge an Partikeln, welche transportiert werden, gesteuert werden. Dies kann den Vorteil erreichen, dass Luft, welche das Laborgerät über einen Ausgang der Vorrichtung zur Erzeugung der Druckdifferenz verlässt, einen vorbestimmten Partikelgrenzwert, insbesondere einen Partikelkonzentrationsgrenzwert, nicht überschreitet.

Das Laborgerät kann eine Steuerung umfassen, welche ausgebildet ist, eine Förderleistung der Vorrichtung zur Erzeugung der Druckdifferenz zu reduzieren, vorzugsweise auf 1% bis 20%, weiter vorzugswiese auf 2% bis 10%, beispielsweise auf 2% bis 5% der Gesamtförderleistung zu reduzieren. Insbesondere kann die Steuerung eine physikalische Kenngröße des Laborgeräts, beispielsweise eine Temperatur, und/oder einen Betriebsmodus des Laborgeräts erfassen, und anhand der physikalischen Kenngröße und/oder des Betriebsmodus eine Förderleistung reduzieren. Mit steigender Gerätetemperatur kann eine Emission von Partikeln in dem Gerät-Innenraum steigen. Beispielsweise ist es möglich, dass das Laborgerät in einem ersten Betriebszustand (beispielsweise in einem normalen Betriebsmodus) mit der reduzierten Förderleistung betrieben wird und in einem zweiten Betriebszustand (beispielsweise in einem Reinigungsmodus, in dem die Temperatur deutlich erhöht wird) mit der vollen Förderleistung betrieben zu werden. So kann mittels der Steuerung der der Förderleistung ein Partikelausstoß des Laborgeräts begrenzt werden, reduziert werden oder zumindest unterhalb eines vorbestimmten Grenzwertes gehalten werden.

Die Vorrichtung zur Erzeugung der Druckdifferenz kann eine maximale Förderleistung aufweisen, welche einem 20-fachen bis 30-fachen Luftwechsel innerhalb einer Stunde entspricht. Entsprechend kann die Vorrichtung zur Erzeugung der Druckdifferenz ausgebildet sein, innerhalb einer Stunde ein Luftvolumen zu fördern, welches einem 20-fachen bis 30-fachen des in dem Gerät-Innenraum enthaltenen Luftvolumen entspricht. Alternativ kann die maximale Förderleistung anhand des Gesamtgerätevolumens des Laborgeräts definiert sein.

Die Vorrichtung zur Erzeugung der Druckdifferenz kann dazu ausgelegt sein, Gas aus dem Gerät-Innenraum in die Umgebung zu fördern. Insbesondere kann die Vorrichtung zur Erzeugung der der Druckdifferenz ausgebildet sein, nach einem initialen Erzeugen eines Unterdrucks eine Luftmenge aus dem Gerät-Innenraum zu transportieren, welche einer nachströmenden Luftmenge durch nichtgedichtete Bereiche des Laborgeräts entspricht oder größer als diese nachströmende Luftmenge ist. Dadurch kann der Unterdruck kontinuierlich in dem Gerät-Innenraum erhalten werden.

Das Laborgerät kann einen Filter aufweisen, der zwischen einem Auslass der Vorrichtung zur Erzeugung der Druckdifferenz und der Umgebung des Laborgerätes angeordnet ist. Der Filter kann insbesondere derart in dem Laborgerät angeordnet sein, dass ein Luftvolumen, welches von der Vorrichtung zur Erzeugung der Druckdifferenz ausgestoßen wird, zumindest teilweise durch den Filter von dem Gerät-Innenraum in die Laboratmosphäre transportiert wird. Vorzugsweise wird dieses Ausstoßvolumen vollständig durch den Filter geleitet. Der Filter kann ausgebildet sein, Partikel aus der den Filter durchströmenden Luft zu filtern, um eine Partikelkonzentration der Luft, welche von dem Laborgerät in die Laboratmosphäre ausgestoßen wird, zu reduzieren.

Es kann insbesondere ein Filter verwendet werden, welcher ausgebildet ist, eine maximale Partikelemissionsrate des Laborgeräts unterhalb eines vorbestimmten Grenzwerts für die Verwendung des Geräts in einem Reinraum zu reduzieren. Vorteilhafterweise erreicht der Filter eine Partikelfilterung, welche den Einsatz des Laborgeräts in einem Reinraum der ISO-Klasse 5 oder besser ermöglicht.

Der Filter kann ein Schwebstofffilter sein. Der Schwebstofffilter kann zur Abscheidung von Schwebstoffen in Abhängigkeit von einem aerodynamischen Durchmesser der Schwebstoffe, respektive Partikel, ausgebildet sein. Partikel, welche dem Luftstrom um Filterfasern des Filters folgen, können an diesen haften bleiben, wenn die Partikel sich den Filterfasern annähern. Ferner können Partikel, welche dem Luftstrom um die Filterfasern aufgrund ihrer Größe nicht folgen können, gegen diese prallen und daran durch einen Aufprall haften bleiben. Partikel mit einem aerodynamischen Durchmesser kleiner als 1 µm können dem Luftstrom nicht folgen und kollidieren beispielsweise durch zufällige Bewegung mit den Filterfasern und bleiben an diesen haften.

Der Filter kann einen Abscheidegrad von mindestens 99%, vorzugsweise von mindestens 99,9%, weiter vorzugsweise von mindestens 99,95%, noch weiter vorzugsweise von mindestens 99,995%, bezogen auf Partikel mit einer Partikelgröße, die am schwersten abzuscheiden sind. Bei diesen Partikeln handelt es sich üblicherweise um Partikel mit einer Partikelgröße im Bereich von ca. 0,1 µm bis ca. 0,3 µm.

Der Filter kann aus einem Fasermaterial sein, beispielsweise aus Glasfaser.

Der Filter kann lösbar befestigt sein. Insbesondere kann der Filter lösbar an dem äußeren Gehäuse befestigt sein. Dadurch kann der Vorteil erreicht werden, dass der Filter bei einer Sättigung mit Partikeln ausgetauscht werden kann. Beispielsweise können Filter zum einmaligen Gebrauch verwendet werden.

Der Filter kann ein austauschbares Filtermaterial umfassen, wobei zur Widerherstellung einer ursprünglichen Filterleistung das Filtermaterial in dem Filter ausgetauscht, gereinigt oder reaktiviert werden kann.

Das Laborgerät kann ein Schutzgitter umfassen, welches an dem Filter angeordnet ist, sodass der Filter durch äußere Beschädigungen geschützt sein kann. Das Schutzgitter kann regelmäßige, insbesondere hexagonale Öffnungen aufweisen. Das Schutzgitter kann einen Teil des äußeren Gehäuses bilden und/oder das Schutzgitter kann von einer Gehäuseoberfläche des äußeren Gehäuses zurückgesetzt angeordnet sein.

Das äußere Gehäuse kann ein Seitengehäuse, ein Rückgehäuse, ein Deckengehäuse, ein Untergehäuse und ein Türgehäuse aufweisen. Das Rückgehäuse und das Türgehäuse können an entgegengesetzten Enden des Laborgerätes angeordnet sein. Die einzelnen Gehäuseteile können untereinander dichtend verbunden sein, um eine Partikelemission an Stoßstellen und/oder Verbindungspunkten zu reduzieren oder zu unterbinden. An verbleibenden Lufteinlässen des äußeren Gehäuses kann mittels der Vorrichtung zur Erzeugung des Unterdrucks in Bezug auf den Umgebungsdruck ein Unterdruck erzeugt werden, sodass an den verbleibenden Lufteinlässen Luft aus der Umgebung in den Gerät-Innenraum strömen kann, um eine Partikelemission zu reduzieren.

Der Filter kann in dem Rückgehäuse angeordnet sein. Dadurch kann der Vorteil erreicht werden, dass die Abluft, welche von dem Laborgerät in die Laboratmosphäre abgegeben wird, entfernt von der Türöffnung des Laborgeräts aus dem Laborgerät austritt. Entsprechend kann eine Wahrscheinlichkeit, dass in dem Laborgerät prozessierte Proben von dem Laborgerät emittierten Partikeln ausgesetzt sind, reduziert sein. Entsprechend kann das Risiko einer Kontamination reduziert werden. Ferner kann die an der Rückseite austretende Abluft effizient einer weiteren Abluftbehandlung, beispielsweise einer Partikelabscheidung und/oder einer Luftabsaugung zugeführt werden. Insbesondere kann der Filter senkrecht zu einer Aufstellebene des Laborgeräts ausgerichtet sein. Dadurch kann eine Tiefe des Rückgehäuseteils reduziert sein, um eine Gesamttiefe des Laborgeräts zu minimieren.

Das Laborgerät kann einen Filterhalter umfassen, an welchem der Filter angeordnet ist, wobei der Filterhalter einen Teil des äußeren Gehäuses bilden und/oder der Filterhalter mit dem äußeren Gehäuse lösbar verbunden sein kann. Der Filterhalter kann insbesondere mit dem Rückgehäuse verschraubt sein.

Der Filter kann an einer Innenseite oder einer Außenseite des äußeren Gehäuses angeordnet sein.

Das Laborgerät kann ein Gesamtvolumen im Bereich von 0,1 m³ bis 2,5 m³, vorzugsweise im Bereich von 0,2 m³ bis 1,0 m³ und weiter vorzugsweise im Bereich von 0,4 m³ bis 0,8 m³ haben. Dadurch kann das Laborgerät auf oder unter Labortischen positioniert werden. Ferner kann das Laborgerät beweglich in einem Labor angeordnet sein.

Das Laborgerät kann ein Inkubator sein.

Das Laborgerät kann eine Zentrifuge sein.

Das Laborgerät kann eine Inkubationskammer aufweisen. Die Funktion der Inkubationskammer kann unabhängig von der Funktion der Vorrichtung zur Erzeugung einer Druckdifferenz in dem Gerät-Innenraum sein. Die Inkubationskammer kann eine Tür aufweisen, welche eine separate Kammertür unabhängig von dem Türgehäuse des Laborgeräts bilden kann. Vorteilhafterweise ist in einem geschlossenen Zustand, in welchem die Kammertür und/oder das Türgehäuse geschlossen sind, die Inkubationskammer fluiddicht, gasdicht und/oder partikeldicht von dem Gerät-Innenraum getrennt.

Das Laborgerät kann einen Kohlenstoffdioxid-(CO₂)-Sensor aufweisen, welcher ausgebildet ist, unabhängig von einer Sensortemperatur, Luftfeuchtigkeit der umgebenden Atmosphäre, dem Sauerstoffgehalt der umgebenden Atmosphäre und einem barometrischen Druck der umgebenden Atmosphäre den CO₂-Gehalt der umgebenden Atmosphäre zu bestimmen. Der CO₂-Sensor kann ein MEMS-Sensor sein. Ferner kann der CO₂-Sensor eine Wiederherstellungszeit von weniger als 5 Minuten aufweisen. Eine Wiederherstellungszeit kann eine Zeitspanne bis zum Erreichen von vorbestimmten Inkubationskammerbedingungen nach einem Öffnen der Kammertür definiert. Der CO₂-Sensor kann in der Inkubationskammer angeordnet sein.

Das Laborgerät kann eine Vorrichtung zum Einstellen des Sauerstoffgehalts in der Inkubationskammer aufweisen. Die Vorrichtung zum Einstellen des Sauerstoffgehalts kann ausgebildet sein, den Sauerstoffgehalt zur Simulation von hypoxischen Bedingungen in einem Bereich von 1 % bis 21 % Sauerstoff zu regeln. Dieser Sauerstoffgehalt kann insbesondere vorteilhaft für primäre Zellen und Anwendungen in der Stammzell- und Embryonenforschung sein.

Die Vorrichtung zum Einstellen des Sauerstoffgehalts kann ausgebildet sein, den Sauerstoffgehalt zur Simulation von hyperoxischen Bedingungen in einem Bereich von 5% bis 90 % Sauerstoff zu regeln. Diese Bedingungen können für Lungengewebe, Netzhautgewebe oder andere empfindliche Gewebetypen vorteilhaft sein.

Das Laborgerät kann eine Vorrichtung zur Regulierung der Luftfeuchtigkeit innerhalb der Inkubationskammer aufweisen. Generell kann das Laborgerät passiv oder aktiv befeuchtet werden. Beispielsweise kann es sich um einen aktiv befeuchteten Inkubator handeln. Beispielsweise kann die Vorrichtung zur Regulierung der Luftfeuchtigkeit einen Verdampferofen oder einen Aerosolgenerator umfassen. Alternativ kann das Laborgerät passiv befeuchtet werden. In einen solchen Fall kann beispielsweise die Luftfeuchtigkeit innerhalb der Inkubationskammer durch ein Verdampfen von Wasser in einer Verdampfungsschale erhöht werden. Allgemein kann die Inkubationskammer eine Kältestelle aufweisen, welche relativ zu den übrigen Innenflächen der Inkubationskammer eine reduzierte Temperatur aufweist, sodass Feuchtigkeit an der Kältestelle kondensiert. Mittels der Kältestelle kann eine Luftfeuchtigkeit innerhalb der Inkubationskammer reduziert werden.

Die Vorrichtung zur Erzeugung der Druckdifferenz und der Filter können ein Partikelausstoßkontrollsystem bilden, welches ausgebildet ist, die Anzahl an von dem Laborgerät ausgestoßenen Partikel zu begrenzen. Insbesondere kann das Laborgerät eine Steuerung aufweisen, welche ausgebildet ist, den Zustand des Filters zu erfassen und eine Förderleistung der Vorrichtung zur Erzeugung der Druckdifferenz an den Zustand des Filters anzupassen. Insbesondere kann bei einer steigenden Partikelemission innerhalb des Gerät-Innenraums eine Sättigung des Filters mit Partikeln durch Reduzierung der Förderleistung unterbunden werden.

Das Laborgerät kann eine Heizvorrichtung aufweisen, welche ausgebildet ist, das Laborgerät zumindest teilweise zu temperieren. Die Heizvorrichtung kann ausgebildet sein, eine Inkubationskammer zur Inkubation von Proben zu temperieren und/oder die Inkubationskammer zur Sterilisation zu temperieren. Vorzugsweise erreicht die Inkubationskammer während der Sterilisation eine Temperatur von 180°C, wobei insbesondere alle inneren Oberflächen der Inkubationskammer für eine vorbestimmte Zeit eine Temperatur von zumindest 180°C erreichen.

Das Laborgerät kann ein inneres Gehäuse aufweisen, welches eine Kammer definiert. Die Kammer kann in dem äußeren Gehäuse angeordnet sein. Vorteilhafterweise können Außenwände der Kammer beabstandet von Innenflächen des äußeren Gehäuses angeordnet sein. Das innere Gehäuse kann entsprechend thermisch isoliert von der Laboratmosphäre sein. Bei einem Inkubator kann die Kammer insbesondere die Inkubationskammer sein.

Das innere Gehäuse kann Seitenwände, eine Rückwand, eine untere Wand, eine Deckenwand, und einen Türabschnitt aufweisen. Die jeweiligen Abschnitte können zu den korrespondierenden Abschnitten des äußeren Gehäuses ausgerichtet sein. Insbesondere kann der Türabschnitt parallel und angrenzend an das Türgehäuse des äußeren Gehäuses angeordnet sein, sodass die Kammer durch den Türabschnitt und das Türgehäuse zugänglich ist.

Zumindest Teile des inneren Gehäuses können aus Metall sein.

Das Metall des inneren Gehäuses kann Kupfer oder Stahl, vorzugsweise elektropolierter nichtrostender Stahl sein.

Die Seitenwände, die Rückwand, die untere Wand und/oder die Deckenwand können aus dem Metall sein.

Zumindest Teile des äußeren Gehäuses (10) können aus Metall sein.

Das Metall kann Stahl sein.

Das Metall kann rostfreier und/oder gebürsteter Stahl und vorzugsweise gebürsteter, rostfreier 304 Stahl sein. Metall und insbesondere rostfreier Stahl können den Vorteil realisieren, dass eine reduzierte Partikelemission bereitgestellt wird, insbesondere bei einer erhöhten Relativtemperatur des Laborgeräts in Bezug auf eine Temperatur der Laboratmosphäre. Ferner können Metalle eine verbesserte Kompatibilität zu standardisierten Reinraumreinigungsverfahren aufweisen, sodass Partikelablagerungen auf den Oberflächen effizient entfernt werden können. Ferner können zumindest eine Auswahl an Oberflächen des Laborgeräts poliert sein, insbesondere elektropoliert sein. Polieren kann eine Ablagerung von Partikeln auf der Oberfläche reduzieren, eine Emission von Partikeln von der Oberfläche reduzieren und/oder ein Entfernen von Partikeln von der Oberfläche erleichtern.

In dem Betriebszustand kann der Druck im Geräte-Innenraum in einem Bereich vorhanden sein, der außen durch das äußere Gehäuse und innen durch das innere Gehäuse begrenzt wird, und dieser Druck kann geringer sein als ein Druck in der Kammer. Vorteilhafterweise ist die Kammer zwar in dem Laborgerät angeordnet aber nicht Teil des Gerät-Innenraums, welcher fluidtechnisch mit der Vorrichtung zur Erzeugung der Druckdifferenz verbunden ist. Typischerweise kann in der Kammer der Umgebungsdruck herrschen. Insbesondere kann das Laborgerät mit einer Vorrichtung ausgestattet sein, die den Druck in der Kammer an den Umgebungsdruck anpasst.

Das Laborgerät kann einen Schaltkasten umfassen, wobei die Vorrichtung zur Druckerzeugung ausgebildet sein kann, einen Unterdruck in dem Schaltkasten zu erzeugen. Vorteilhafterweise ist der Schaltkasten im Rückbereich gebildet. Die Vorrichtung zur Erzeugung der Druckdifferenz kann in dem Schaltkasten angeordnet sein. Der Schaltkasten kann durch eine Wand vom übrigen Geräte-Innenraum getrennt sein, wobei die Wand eine Wandfläche aufweist und wobei die Wand mindestens eine Öffnung aufweist. Die entsprechenden Bereiche können also mittels der mindestens einen Öffnung fluidisch miteinander verbunden sein.

Die Vorrichtung zur Erzeugung des Differenzdrucks kann in einem Rückbereich angeordnet sein, der durch die Rückwand und das Rückgehäuse begrenzt wird. Seitenbereiche des Geräte-Innenraums können durch die Seitenwände und die Seitengehäuse begrenzt werden

Die mindestens eine Öffnung kann eine Gesamt-Querschnittsfläche (also die Summe der Querschnittsflächen der einen oder mehr Öffnungen) aufweisen, die im Bereich von 0,1% bis 20% der Wandfläche, vorzugsweise im Bereich im Bereich von 0,5 % bis 10% der Wandfläche, weiter bevorzugt im Bereich von 1% bis 5% der Wandfläche liegt. Die mindestens eine Öffnung kann in Abhängigkeit von einer Förderleistung der Vorrichtung zur Erzeugung des Differenzdrucks einen vorbestimmten Volumenstrom von dem Seitengehäuse zu dem Rückgehäuse erzeugen. Dadurch kann ebenfalls der Partikeleintrag von dem Seitenbereich in den Rückbereich und schlussendlich in den Filter vorbestimmt werden. Die Öffnung kann durch eine Mehrzahl von Durchlässen gebildet sein. Insbesondere kann die Öffnung eine Mehrzahl von Durchgangslöchern umfassen.

Das Laborgerät kann ausgebildet sein, einen Unterdruck in einem Türbereich zu erzeugen, der durch das Türgehäuse und den Türabschnitt begrenzt wird. Der Türgehäuse kann mit den Seitenbereichen und/oder mit dem Rückbereich fluidtechnisch gekoppelt sein, um einen Luftvolumenstrom von dem Türgehäuse zu der Vorrichtung zur Erzeugung der Druckdifferenz zu erzeugen.

Das Laborgerät kann mindestens einen Schlauch umfassen, der die Vorrichtung zur Erzeugung des Differenzdruckes fluidisch mit mindestens einem anderen Bereich verbindet.

Der mindestens eine Schlauch kann den Türbereich und die Vorrichtung zur Erzeugung des Differenzdrucks fluidisch verbinden. Beispielsweise kann der Schlauch an die Pumpe angeschlossen sein. Ein Schlauchende kann an einer Position des Türgehäuses angeordnet, welche eine erhöhte Partikelemission aufweisen kann, sodass die Partikel durch den Schlauch zu der Vorrichtung zur Erzeugung des Differenzdrucks transportiert werden können.

Der mindestens eine Schlauch kann die Vorrichtung zur Erzeugung der Druckdifferenz fluidisch mit einem Vorderbereich verbinden, der durch die Seitengehäuse, die Seitenwände, das Deckengehäuse, die Deckenwand, das Untergehäuse und die untere Wand begrenzt wird und an den Türabschnitt angrenzt.

Der mindestens eine Schlauch kann zumindest eine Abzweigung umfassen, an welcher zumindest zwei Schlauchsegmente angeschlossen sind, die jeweils eine Schlauchöffnung aufweisen.

Die Schlauchöffnungen der Schlauchsegmente können beabstandet voneinander angeordnet sein.

Die Schlauchöffnungen können in dem Türbereich angeordnet sein. Entsprechend können mehrere Stellen innerhalb des Türbereichs mittels des Schlauchs zur Absaugung von Partikeln erreicht werden. Beispielsweise kann ein Schlauch perforiert sein, um Partikel aus der Umgebung des Schlauchs abzuleiten. Hierzu kann ein einzelnes Schlauchsegment in dem Türgehäuse angeordnet sein.

Der Schlauch kann zumindest teilweise in einem Hohlverbindungselement angeordnet sein, wobei das Hohlverbindungselement ausgebildet ist, einen Verbindungskanal zwischen dem Türgehäuse und dem Rückgehäuse, und/oder dem Seitgengehäuse und/oder dem Deckengehäuse zu realisieren.

Der mindestens eine Schlauch kann aus einem Material sein, das eine Temperaturbeständigkeit bis mindestens 200° C, vorzugsweise bis mindestens 220 ° C hat.

Der mindestens eine Schlauch kann aus Silikon sein.

Das Laborgerät kann einen Strömungskanal aufweisen, welcher die Vorrichtung zur Erzeugung der Druckdifferenz mit einer Auslassöffnung in dem äußeren Gehäuse verbindet. Dies kann den Vorteil erreichen, dass die Vorrichtung zur Erzeugung der Druckdifferenz räumlich getrennt von der Auslassöffnung angeordnet werden kann. Ferner können Bauteile, welche einen Kühlluftstrom benötigen, insbesondere elektronische Bauteile, in dem Strömungskanal angeordnet sein. Ein Teil des Strömungskanals kann durch den Schaltkasten gebildet sein. Der Strömungskanal kann das innere Gehäuse und das äußere Gehäuse verbinden, respektive durch das innere Gehäuse und das äußere Gehäuse begrenzt sein.

Der Filter kann in dem Strömungskanal und/oder an der Auslassöffnung angeordnet sein. Entsprechend kann der Vorteil erreicht werden, dass das Luftvolumen, welches mittels der Vorrichtung zur Erzeugung der Druckdifferenz durch den Strömungskanal strömt, mit Austritt aus dem Laborgerät eine reduzierte Partikelanzahl aufweist.

Die Vorrichtung zur Erzeugung der Druckdifferenz kann an dem Strömungskanal angeordnet sein. Insbesondere kann ein Auslass einer Pumpe der Vorrichtung zur Erzeugung der Druckdifferenz in dem Strömungskanal angeordnet sein.

Die Vorrichtung zur Erzeugung der Druckdifferenz kann eine Auslassöffnung aufweisen, welche mit dem Strömungskanal verbunden ist. So kann Luft in dem Gerät-Innenraum angesaugt werden und durch den Auslassabschnitt kann Luft aus dem Laborgerät in die Laboratmosphäre abgeblasen werden.

Der Filter kann den äußeren Abschnitt des Strömungskanals abschließen, sodass ein Luftvolumen, welches durch den Strömungskanal strömt, vollständig den Filter passiert.

Das Laborgerät kann mindestens ein thermisches Isolationsbauteil umfassen, welches zumindest teilweise aus einem thermischen Isolationsmaterial besteht. Das thermische Isolationsmaterial ist vorteilhafterweise flexibel und/oder kompressibel, insbesondere kann Glaswolle bzw. Mineralwolle verwendet werden. Dadurch kann der Vorteil erreicht werden, dass bestehende Hohlräume eine hohe Füllungsdichte mit Isolationsmaterial aufweisen, sodass das Gerät (beispielsweise eine Inkubationskammer im Gerät) geeignet von der äußeren Atmosphäre isoliert werden. Das Isolationsmaterial kann eine Temperaturbeständigkeit von mindestens 200°C, bevorzugt von mindestens 220°C aufweisen.

Das mindestens thermische Isolationsbauteil kann zumindest teilweise aus einem thermischen Isolationsmaterial bestehen.

Das mindestens eine thermische Isolationsbauteil kann in einem Zwischenraum zwischen dem äußeren Gehäuse und dem inneren Gehäuse angeordnet sein. Dadurch kann insbesondere eine thermische Isolation verbessert werden, sodass innerhalb der Kammer eine höhere Temperaturstabilität erreicht werden kann. Vorteilhafterweise ist an allen Seitendes inneren Gehäuses eine flächig ausgebildetes Isolationsbauteil vorgesehen.

Das mindestens eine thermische Isolationsbauteil kann zwischen den Seitenwänden und dem Seitengehäuse, zwischen der Rückwand und dem Rückgehäuse, zwischen der unteren Wand und dem Untergehäuse, zwischen der Deckenwand und dem Deckengehäuse und/oder zwischen dem Türabschnitt und dem Türgehäuse angeordnet sein.

Das mindestens eine thermische Isolationsbauteil kann eine Abschlussschicht aufweisen, welche das mindestens eine thermische Isolationsbauteil versiegelt. Dadurch kann der Vorteil erreicht werden, dass eine Partikelemission des thermischen Isolationsmaterials reduziert ist. Insbesondere ein Anstieg der Partikelemission mit steigender Gerätetemperatur kann reduziert sein, sodass nicht nur insgesamt weniger Partikel von dem thermischen Isolationsbauteil freigesetzt werden, sondern auch eine Erhöhung der Partikelemission mit steigender Temperatur verlangsamt werden kann. Vorteilhafterweise umschließt die Abschlussschicht das thermische Isolationsmaterial vollständig.

Die Abschlussschicht kann eine Folie umfassen. Die Folie kann insbesondere eine Partikelemissionsschutzschicht bilden, welche an dem Isolationsmaterial zur Anlage kommt und/oder mit diesem verklebt ist. Die Folie kann ein abgeschlossenes Volumen bilden, in welchem das thermische Isolationsmaterial angeordnet ist, um das thermische Isolationsmaterial von der Atmosphäre in dem Gerät-Innenraum, respektive der Laboratmosphäre abzuschirmen.

Die Folie kann eine Temperaturbeständigkeit bis mindestens 200 °C, vorzugsweise bis mindestens 220 °C haben. Weiterbevorzugt kann eine Temperaturbeständigkeit der Folie in dem Bereich von 200 °C bis 300 °C liegen. Dadurch kann der Vorteil erreicht werden, dass während eines Betriebsmodus des Laborgeräts mit einer entsprechend hohen Gerätetemperatur, die Folie weiterhin eine emissionsreduzierende Wirkung aufweist. Insbesondere kann eine Emission von Partikeln von der Folie selbst reduziert sein. Vorteilhafterweise weist die Folie eine temperaturunabhängige Partikelemissionsrate auf oder die Partikelemissionsrate ist zumindest nur geringfügig abhängig von einer Temperatur der Folie.

Die Folie kann flexibel und/oder emissionsarm ausgebildet sein. Dadurch kann der Vorteil einer effizienten Verarbeitung bei einem Zusammenfügen von dem thermischem Isolationsmaterial und der Folie erreicht werden. Ferner ist die Folie der Gerätatmosphäre und entsprechend dem Differenzdruck ausgesetzt, sodass Partikelemissionen der Folie auch Teil einer Gesamtpartikelemission des Laborgeräts sind. Je geringer die Partikelemission der Folie selbst, desto geringer kann eine Gesamtpartikelemission des Laborgeräts sein. Die Folie kann insbesondere undurchlässig oder emissionsreduzierend für Partikel des thermischen Isolationsmaterials sein.

Die Folie kann eine Klebeschicht umfassen, welche ausgebildet ist, überlappende Lagen der Folie zu verkleben, um das thermische Isolationsbauteil zu versiegeln, insbesondere partikeldicht oder die Partikelemission-reduzierend zu versiegeln. Dadurch kann der Vorteil erreicht werden, dass in Überlappungsbereichen der Folie eine gleichbleibend hohe Reduzierung der Partikelemission erreicht werden kann. Die Klebeschicht kann ebenso eine Temperaturstabilität von mindestens zu 200°C, vorzugsweise von mindestens 220°C aufweisen.

Die Klebeschicht kann ausgebildet sein, die Abschlussschicht mit dem thermischen Isolationsmaterial zu verkleben. Die Klebeschicht kann dauerhaft flexibel ausgebildet sein, um der thermisch-bedingten Ausdehnung der Folie zu folgen. Mit der Verbindung der Klebeschicht mit einer Oberfläche des thermischen Isolationsmaterials kann eine Partikelemission des thermischen Isolationsmaterials an der entsprechenden Oberfläche reduziert sein. Die Klebeschicht kann die Funktion eines Partikelfilters, respektive einer Partikelfalle aufweisen. Dies Wirkung kann mit einer Sättigung der Klebeschicht mit Partikeln reduziert sein.

Das thermische Isolationsmaterial kann in die Abschlussschicht eingeschlagen sein. Ferner können Überlappungsbereiche der Folie mit einem weiteren Folienabschnitt überdeckend verklebt sein. Dadurch kann der Vorteil erreicht werden, dass an Schnittkanten eine zu den unverklebten Bereichen der Folie ähnliche Partikelemissionsreduktion bewirkt werden kann. Insbesondere können auftretende Imperfektionen in der ersten Verklebung der Folie durch die Verklebung des überdeckenden Folienabschnitts ausgeglichen werden.

Die Folie kann eine Kunststofffolie sein, welche vorzugsweise ein Polymer enthält.

Die Folie kann Polyamid umfassen. Dadurch kann der Vorteil erreicht werden, dass die Abschlussschicht wasserdicht, formbeständig, reißfest, hoch elastisch und langlebig ist.

Die Abschlussschicht kann eine Folie umfassen, welche mittels eines Polyamidband verklebt ist. Das Polyamidband kann eine Silikonklebeschicht aufweisen, welche ausgebildet ist, Folienabschnitte der Abschlussschicht zu verkleben und/oder die Abschlussschicht mit dem thermischen Isolationsbauteil zu verbinden.

Die Klebeschicht kann eine Silikonklebstoffschicht sein. Die Silikonklebstoffschicht kann eine Schicht der Folie bilden. Insbesondere kann die Folie zweischichtig aufgebaut sein, wobei eine erste Schicht eine Polyamidschicht ist und eine zweite Schicht eine Silikonschicht ist.

Das Laborgerät kann eine Steuereinheit umfassen, welche ausgebildet ist, eine Förderleistung der Vorrichtung zur Erzeugung der Druckdifferenz an einen Betriebsmodus des Laborgeräts anzupassen. Dadurch kann insbesondere der Vorteil erreicht werden, dass die Förderleistung an eine Partikelemissionsrate in dem Gerät-Innenraum, respektive durch die Bauteile des Laborgeräts angepasst werden kann.

Die Steuereinheit kann einen ersten Betriebsmodus aufweisen und die Vorrichtung zur Erzeugung des Differenzdrucks kann ein Gebläse und eine Pumpe umfassen, wobei in dem ersten Betriebsmodus ausschließlich das Gebläse aktiv ist.

Die Steuereinheit kann einen zweiten Betriebsmodus aufweisen, wobei in dem zweiten Betriebsmodus sowohl das Gebläse als auch die Pumpe aktiv sind. Dadurch kann der Vorteil erreicht werden, dass mit der Pumpe Partikel in Abschnitten des Gerät-Innenraums zu dem Filter geführt werden können, welche durch das Gebläse allein einem unzureichend niedrigen Luftstrom ausgesetzt sind. Vorteilhafterweise kann mittels eines Schlauchsystems, welches Schlauchöffnungen aufweist, welche in Bereichen mit erhöhter Partikelemission angeordnet sind, ein effizienter Partikeltransport zu der Vorrichtung zur Erzeugung der Druckdifferenz und/oder zu dem Filter realisiert werden. Insbesondere können Gehäuseabschnitte mit zunehmendem Abstand von der Vorrichtung zur Erzeugung der Druckdifferenz einen unzureichenden Luftstrom in Richtung der der Vorrichtung zur Erzeugung der Druckdifferenz aufweisen, sodass ein Transportvolumen von Partikeln aus diesem Bereich reduziert sein kann.

Vorteilhafterweise ist zumindest eine Schlauchöffnung in dem Türbereich angeordnet, um Partikel aus dem Türbereich zu entfernen. Insbesondere kann der Türbereich mittels des Schlauchs mit der Vorrichtung zur Erzeugung der Druckdifferenz fluidtechnisch gekoppelt sein.

Die Steuereinheit kann ausgebildet sein, bei Erreichen eines Temperaturgrenzwerts von dem ersten Betriebsmodus in den zweiten Betriebsmodus zu wechseln. Die Gerätetemperatur kann ein Maß für eine Partikelemissionsrate des Laborgeräts sein. Entsprechend kann der Vorteil erreicht werden, dass eine Förderleistung der Vorrichtung zur Erzeugung der Druckdifferenz mit der Partikelemissionsrate des Laborgeräts skaliert. Insbesondere können Gehäuseteile, welche eine besonders temperaturabhängige Partikelemissionsrate aufweisen oder in dem ersten Betriebsmodus einen geringen Volumenstrom erfahren, in dem zweiten Betriebsmodus strömungstechnisch effizienter an die Vorrichtung zur Erzeugung der Druckdifferenz angebunden sein.

Die Förderleistung kann in Abhängigkeit von einer Temperatur des Laborgeräts oder eines Teils des Laborgeräts angepasst werden. Hierbei kann bei Verwendung einer Pumpe und eines Gebläses selektiv jeweils eine Förderleistung angepasst werden, um einen hinreichenden Partikeltransport aus den Gehäusebereichen, welche hauptsächlich von dem Gebläse erreicht werden, und aus Bereichen welche hauptsächlich von der Pumpe erreicht werden, zu gewährleisten. Ferner kann die Förderleistung bei einer steigenden Partikelemission gesteigert werden, beispielsweise um einen vorgegebenen Emissionswert nicht zu überschreiten.

Eine Förderleistung des Gebläses kann konstant sein und eine Förderleistung der Pumpe kann anpassbar sein, um eine Gesamtförderleistung der Vorrichtung zur Erzeugung der Druckdifferenz zu erhöhen.

Die Pumpe kann zusätzlich zu dem Gebläse geschaltet werden, wenn eine vorbestimmte Temperatur des Laborgeräts erreicht wird, um eine Förderleistung zu erhöhen.

Die Vorrichtung zur Erzeugung der Druckdifferenz kann ausgebildet sein, Partikel aus dem Innenraum des äußeren Gehäuses, insbesondere dem Türbereich, abzusaugen.

Die Vorrichtung zur Druckerzeugung kann eine minimale Förderleistung aufweisen, um eine gerichtete Luftströmung von der Umgebung des Laborgeräts in das äußere Gehäuse zu generieren. Dadurch kann sichergestellt werden, dass stets ein Unterdruck in dem Gerät-Innenraum erzeugt wird. Ferner kann bei einer minimalen Förderrate der Partikelausstoß des Laborgeräts minimal sein.

Der Schaltkasten kann ein Schaltkastenbauteil umfassen. Die Vorrichtung zur Erzeugung des Differenzdrucks kann ausgebildet sein, einen Luftstrom in dem Schaltkasten zu erzeugen und den Luftstrom in Abhängigkeit einer Bauteiltemperatur des Schaltkastenbauteils zu steuern. Dadurch können Bauteile in dem Schaltkasten durch den von der Vorrichtung zur Erzeugung des Differenzdrucks erzeugten Luftstrom gekühlt werden.

Das Schaltkastenbauteil kann ein Kühlelement, insbesondere ein Kühlwinkel, sein.

Das Kühlelement kann zumindest teilweise an dem inneren Gehäuse angeordnet sein. Vorteilhafterweise ist das Kühlelement mit einer Gehäusewand des inneren Gehäuses thermisch gekoppelt, um an einer Innenwand des inneren Gehäuses eine Fläche mit reduzierter Temperatur zu erzeugen. An dieser Fläche kann Feuchtigkeit kondensieren.

Das Laborgerät kann eine Schließvorrichtung umfassen, welche ausgebildet ist, das Türgehäuse mit einem weiteren Teil des äußeren Gehäuses verschließbar zu verbinden. Die Schließvorrichtung kann luftgefüllt sein. Insbesondere kann die luftgefüllte Schließvorrichtung an einem Anschlag des Türgehäuses an dem Seitengehäuse oder Deckengehäuse, vorzugsweise an dem Untergehäuse angeordnet sein. Ferner kann eine Schlauchöffnung der Vorrichtung zur Erzeugung des Differenzdrucks an oder in der luftgefüllten Schließvorrichtung angeordnet sein.

Das Laborgerät kann ausgebildet sein, in der Schließvorrichtung einen Unterdruck zu erzeugen. Entsprechend kann eine Emission von Partikeln ausgehend von der luftgefüllten Schließvorrichtung reduziert sein.

Das äußere Gehäuse kann zumindest teilweise luftdicht ausgebildet sein. Insbesondere kann das äußere Gehäuse derart abgedichtet sein, dass bestehende Gehäuseöffnungen in einem Wirkbereich der Vorrichtung zur Erzeugung des Differenzdrucks liegen, um einen Partikelaustritt in diesen Bereichen zu unterbinden oder zumindest zu reduzieren.

Die thermischen Isolationsbauteile können derart in dem Gerät-Innenraum angeordnet sein, dass zwischen den thermischen Isolationsbauteilen und einer Innenwand des äußeren Gehäuses und/oder zwischen den thermischen Isolationsbauteilen und einer Außenwand des inneren Gehäuses Luftkanäle gebildet sind. Insbesondere kann ein Netzwerk an Luftkanälen bestehen, wobei die Vorrichtung zur Erzeugung des Differenzdrucks ausgebildet ist, in diesen Luftkanälen einen Unterdruck, respektive eine Luftströmung zu erzeugen, um Partikel in Richtung der Vorrichtung zur Erzeugung des Differenzdrucks zu transportieren. Die Luftkanäle können in dem jeweiligen Gehäuseabschnitt einen Strömungsquerschnitt bilden, wobei die Summe der durch die Vorrichtung zur Erzeugung des Differenzdrucks erreichbaren Luftkanäle einen Gesamtströmungsquerschnitt bilden, auf welchen sich die Förderleistung der Vorrichtung zur Erzeugung des Differenzdrucks verteilt. Zusätzlich können Schläuche welche mit der Pumpe verbunden sind, einen Teil des Gesamtströmungsquerschnitts bilden.

Die Luftkanäle können Teil des Strömungskanals sein.

Die Erfindung betrifft auch die Verwendung des beschriebenen Laborgerätes in einem Reinraum.

Die Verwendung kann zur Inkubation von Organismen, Zellen, Bakterien und/oder Viren sein.

Die Verwendung kann zur Sterilisation eines inneren Gehäuses des Laborgerätes sein.

Die Erfindung wird auch anhand der folgenden nummerierten Ausführungsformen weiter beschrieben.

Nachfolgend sind Geräteausführungsformen genannt. Diese Ausführungsformen werden mit dem Buchstaben "G", gefolgt von einer Zahl, abgekürzt. Wann immer im Folgenden auf "Geräteausführungsform" Bezug genommen wird, sind diese Ausführungsformen gemeint.
G1. Laborgerät (1), wobei das Laborgerät (1) ein äußeres Gehäuse (10) aufweist, das einen Gerät-Innenraum definiert, wobei das Laborgerät (1) ausgelegt ist, einen Betriebszustand einzunehmen, bei dem ein Druck in dem Gerät-Innenraum geringer ist als ein Umgebungsdruck in der Umgebung des Laborgerätes (1).
G2. Das Laborgerät (1) nach der vorstehenden Ausführungsform, wobei eine Differenz zwischen dem Umgebungsdruck und dem Druck im Bereich von 1 Pa bis 1000 Pa, bevorzugt im Bereich von 2 Pa bis 500 Pa, weiter bevorzugt im Bereich von 5 Pa bis 400 Pa liegt.
G3. Das Laborgerät (1) nach einer der vorstehenden Ausführungsformen, wobei das Laborgerät (1) eine Vorrichtung zur Erzeugung der Druckdifferenz aufweist.
G4. Das Laborgerät (1) nach der vorstehenden Ausführungsform, wobei die Vorrichtung zur Erzeugung der Druckdifferenz ein Gebläse (30) und/oder eine Pumpe (32) umfasst.
G5. Das Laborgerät (1) nach einer der vorstehenden Ausführungsformen mit den Merkmalen der Ausführungsform G3, wobei die Vorrichtung zur Erzeugung der Druckdifferenz ausgelegt ist, eine Förderleistung im Bereich vom 10-fachen bis 40-fachen eines Gesamtvolumens des Laborgerätes pro Stunde zu erzielen, vorzugsweise im Bereich vom 15-fachen bis 30-fachen des Gesamtvolumens des Laborgerätes pro Stunde zu erzielen.
G6. Das Laborgerät (1) nach einer der vorstehenden Ausführungsformen mit den Merkmalen der Ausführungsform G3, wobei das Laborgerät (1) eine Steuerung umfasst, welche ausgebildet ist, eine Förderleistung der Vorrichtung zur Erzeugung der Druckdifferenz zu reduzieren, vorzugsweise auf 1% bis 20%, weiter vorzugswiese auf 2% bis 10%, beispielsweise auf 2% bis 5% einer Gesamtförderleistung der Vorrichtung zur Erzeugung der Druckdifferenz zu reduzieren.
G7. Das Laborgerät (1) nach einer der vorstehenden Ausführungsformen mit den Merkmalen der Ausführungsform G3, wobei die Vorrichtung zur Erzeugung der Druckdifferenz dazu ausgelegt ist, Gas aus dem Gerät-Innenraum in die Umgebung des Laborgeräts (1) zu fördern.
G8. Das Laborgerät (1) nach der vorstehenden Ausführungsform, wobei das Laborgerät (1) einen Filter (50) aufweist, der zwischen einem Auslass der Vorrichtung zur Erzeugung der Druckdifferenz und der Umgebung des Laborgerätes (1) angeordnet ist.
G9. Das Laborgerät (1) nach der vorstehenden Ausführungsform, wobei der Filter (50) ein Schwebstofffilter ist.
G10. Das Laborgerät (1) nach einer der vorstehenden Ausführungsformen mit den Merkmalen der Ausführungsform G8, wobei der Filter (50) lösbar befestigt ist.
G11. Das Laborgerät (1) nach einer der vorstehenden Ausführungsformen mit den Merkmalen der Ausführungsform G8, wobei der Filter (50) ein austauschbares Filtermaterial umfasst.
G12. Das Laborgerät (1) nach einer der vorstehenden Ausführungsformen mit den Merkmalen der Ausführungsform G8, wobei das Laborgerät (1) ein Schutzgitter (39) umfasst, welches an dem Filter (50) angeordnet ist.
G13. Das Laborgerät (1) nach einer der vorstehenden Ausführungsformen, wobei das äußere Gehäuse (10)
   ein Seitengehäuse (12),
   ein Rückgehäuse (18),
   ein Deckengehäuse (14),
   ein Untergehäuse (16) und
   ein Türgehäuse (19) aufweist,
   wobei das Rückgehäuse (18) und das Türgehäuse (19) an entgegengesetzten Enden des Laborgerätes (1) angeordnet sind.
G14. Das Laborgerät (1) nach der vorstehenden Ausführungsform mit den Merkmalen der Ausführungsform G8, wobei der Filter (50) in dem Rückgehäuse (18) angeordnet ist.
G15. Das Laborgerät (1) nach einer der vorstehenden Ausführungsformen mit den Merkmalen der Ausführungsform G13, wobei das Laborgerät (1) einen Filterhalter (37) umfasst, welcher einen Teil des äußeren Gehäuses (10) bildet und/oder wobei der Filterhalter (37) mit dem äußeren Gehäuse (10) lösbar verbunden ist.
G16. Das Laborgerät (1) nach einer der vorstehenden Ausführungsformen mit den Merkmalen der Ausführungsformen G8 und G13, wobei der Filter (50) an einer Innenseite oder einer Außenseite des äußeren Gehäuses (10) angeordnet ist.
G17. Das Laborgerät (1) nach einer der vorstehenden Ausführungsformen, wobei das Laborgerät ein Gesamtvolumen im Bereich von 0,1 m³ bis 2,5 m³, vorzugsweise im Bereich von 0,2 m³ bis 1,0 m³ und weiter vorzugsweise im Bereich von 0,4 m³ bis 0,8 m³ hat.
G18. Das Laborgerät (1) nach einer der vorstehenden Ausführungsformen, wobei das Laborgerät (1) ein Inkubator ist.
G19. Das Laborgerät (1) nach einer der Ausführungsformen G1 bis G17, wobei das Laborgerät (1) eine Zentrifuge ist.
G20. Das Laborgerät (1) nach einer der vorstehenden Ausführungsformen mit den Merkmalen der Ausführungsform G18, wobei das Laborgerät (1) eine Inkubationskammer aufweist.
G21. Das Laborgerät (1) nach einer der vorstehenden Ausführungsformen mit Merkmalen der Ausführungsformen G3 und G8, wobei die Vorrichtung zur Erzeugung der Druckdifferenz und der Filter (50) ein Partikelausstoßkontrollsystem bilden, welches ausgebildet ist, die Anzahl an von dem Laborgerät (1) ausgestoßenen Partikel zu begrenzen.
G22. Das Laborgerät (1) nach einer der vorstehenden Ausführungsformen, wobei das Laborgerät (1) eine Heizvorrichtung aufweist.
G23. Das Laborgerät (1) nach einer der vorstehenden Ausführungsformen, wobei das Laborgerät (1) ein inneres Gehäuse (20) aufweist, welches eine Kammer definiert.
G24. Das Laborgerät (1) nach der vorstehenden Ausführungsform, wobei das innere Gehäuse (20)
   Seitenwände (22),
   eine Rückwand (28),
   eine untere Wand (26),
   eine Deckenwand (24) und
   einen Türabschnitt (29) aufweist.
G25. Das Laborgerät (1) nach einer der 2 vorstehenden Ausführungsformen, wobei zumindest Teile des inneren Gehäuses (20) aus Metall sind.
G26. Das Laborgerät (1) nach einer der vorstehenden Ausführungsformen mit den Merkmalen der Ausführungsform G22, umfassend eine Vorrichtung zur Regelung der Luftfeuchtigkeit in der Kammer.
G27. Das Laborgerät (1) nach einer der vorstehenden Ausführungsformen mit den Merkmalen der Ausführungsform G25, wobei das Metall des inneren Gehäuses (20) Kupfer oder Stahl, vorzugsweise elektropolierter nichtrostender Stahl ist.
G28. Das Laborgerät (1) nach einer der vorstehenden Ausführungsformen mit den Merkmalen der Ausführungsformen G24 und G25, wobei die Seitenwände (22), die Rückwand (28), die untere Wand (26) und die Deckenwand (24) aus dem Metall sind.
G29. Das Laborgerät (1) nach einer der vorstehenden Ausführungsformen, wobei zumindest Teile des äußeren Gehäuses (10) aus Metall sind.
G30. Das Laborgerät (1) nach der vorstehenden Ausführungsform, wobei das Metall Stahl ist.
G31. Das Laborgerät (1) nach der vorstehenden Ausführungsform, wobei das Metall rostfreier und/oder gebürsteter Stahl und vorzugsweise gebürsteter, rostfreier 304 Stahl ist.
G32. Das Laborgerät (1) nach einer der vorstehenden Ausführungsformen mit den Merkmalen der Ausführungsformen G3 und G23, wobei in dem Betriebszustand der Druck im Geräte-Innenraum in einem Bereich vorhanden ist, der außen durch das äußere Gehäuse (10) und innen durch das innere Gehäuse (20) begrenzt wird, und wobei dieser Druck geringer ist als ein Druck in der Kammer.
G33. Das Laborgerät (1) nach einer der vorstehenden Ausführungsformen mit den Merkmalen der Ausführungsform G3, wobei das Laborgerät (1) einen Schaltkasten umfasst, wobei die Vorrichtung zur Erzeugung des Differenzdrucks ausgebildet ist, einen Unterdruck in dem Schaltkasten zu erzeugen, wobei der Schaltkasten durch eine Wand vom übrigen Geräte-Innenraum getrennt ist, wobei die Wand eine Wandfläche aufweist und wobei die Wand mindestens eine Öffnung aufweist.
G34. Das Laborgerät (1) nach einer der vorstehenden Ausführungsformen mit den Merkmalen der Ausführungsformen G3, G13 und G24, wobei die Vorrichtung zur Erzeugung des Differenzdrucks in einem Rückbereich angeordnet ist, der durch die Rückwand (28) und das Rückgehäuse (18) begrenzt wird, und wobei Seitenbereiche des Geräte-Innenraums durch die Seitenwände (22) und die Seitengehäuse (12) begrenzt werden.
G35. Das Laborgerät (1) nach einer der vorstehenden Ausführungsformen mit den Merkmalen der Ausführungsform G33, wobei die mindestens Öffnung eine Gesamt-Querschnittsfläche aufweist, die im Bereich von 0,1% bis 20% der Wandfläche, vorzugsweise im Bereich im Bereich von 0,5 % bis 10% der Wandfläche, weiter bevorzugt im Bereich von 1% bis 5% der Wandfläche liegt, und wobei die mindestens eine Öffnung vorzugsweise in Abhängigkeit von einer Förderleistung der Vorrichtung zur Erzeugung des Differenzdrucks einen vorbestimmten Volumenstrom von dem Seitengehäuse (12) zu dem Rückgehäuse (18) erzeugt.
G36. Das Laborgerät (1) nach einer der vorstehenden Ausführungsformen mit den Merkmalen der Ausführungsformen G3, G13 und G24, wobei das Laborgerät (1) ausgebildet ist, einen Unterdruck in einem Türbereich zu erzeugen, der durch das Türgehäuse (19) und den Türabschnitt (29) begrenzt wird.
G37. Das Laborgerät (1) nach einer der vorstehenden Ausführungsformen mit den Merkmalen der Ausführungsform G3, wobei das Laborgerät (1) mindestens einen Schlauch umfasst, der die Vorrichtung zur Erzeugung des Differenzdruckes fluidisch mit mindestens einem anderen Bereich verbindet.
G38. Das Laborgerät (1) nach der vorstehenden Ausführungsform und mit den Merkmalen des Ausführungsform G36, wobei der mindestens eine Schlauch den Türbereich und die Vorrichtung zur Erzeugung des Differenzdrucks fluidisch verbindet.
G39. Das Laborgerät (1) nach einer der vorstehenden Ausführungsformen mit den Merkmalen der Ausführungsformen G13, G24 und G37, wobei der mindestens eine Schlauch die Vorrichtung zur Erzeugung der Druckdifferenz fluidisch mit einem Vorderbereich verbindet, der durch die Seitengehäuse (12), die Seitenwände (22), das Deckengehäuse (14), die Deckenwand (24), das Untergehäuse (16) und die untere Wand (26) begrenzt wird und an den Türabschnitt (29) angrenzt.
G40. Das Laborgerät (1) nach einer der vorstehenden Ausführungsformen mit den Merkmalen der Ausführungsformen G37, wobei der mindestens eine Schlauch zumindest eine Abzweigung umfasst, an welcher zumindest zwei Schlauchsegmente angeschlossen sind, die jeweils mindestens eine Schlauchöffnung aufweisen.
G41. Das Laborgerät (1) nach der vorstehenden Ausführungsform, wobei die Schlauchöffnungen der zumindest zwei Schlauchsegmente beabstandet voneinander angeordnet sind.
G42. Das Laborgerät (1) nach der vorstehenden Ausführungsform und mit den Merkmalen der Ausführungsform G36, wobei die Schlauchöffnungen in dem Türbereich angeordnet sind.
G43. Das Laborgerät nach einer der vorstehenden Ausführungsformen mit den Merkmalen der Ausführungsform G37, wobei der mindestens eine Schlauch aus einem Material ist, das eine Temperaturbeständigkeit bis mindestens 200° C, vorzugsweise bis mindestens 220 ° C hat.
G44. Das Laborgerät nach einer der vorstehenden Ausführungsformen mit den Merkmalen der Ausführungsform G37, wobei der mindestens eine Schlauch aus Silikon ist.
G45. Das Laborgerät (1) nach einer vorstehenden Ausführungsformen mit den Merkmalen der Ausführungsform G3, wobei das Laborgerät (1) einen Strömungskanal aufweist, welcher die Vorrichtung zur Druckerzeugung mit einer Auslassöffnung in dem äußeren Gehäuse (10) verbindet.
G46. Das Laborgerät (1) nach einer der vorstehenden Ausführungsformen mit den Merkmalen der Ausführungsformen G8 und G45, wobei der Filter (50) in dem Strömungskanal und/oder an der Auslassöffnung angeordnet ist.
G47. Das Laborgerät (1) nach einer der vorstehenden Ausführungsformen mit den Merkmalen der Ausführungsformen G3 und G45, wobei die Vorrichtung zur Erzeugung der Druckdifferenz an dem Strömungskanal angeordnet ist.
G48. Das Laborgerät (1) nach einer der vorstehenden Ausführungsformen mit den Merkmalen der Ausführungsformen G3 und G45, wobei die Vorrichtung zur Erzeugung der Druckdifferenz eine Auslassöffnung aufweist, welche mit dem Strömungskanal verbunden ist.
G49. Das Laborgerät (1) nach einer der vorstehenden Ausführungsformen mit den Merkmalen der Ausführungsformen G8 und G45, wobei der Filter (20) den äußeren Abschnitt des Strömungskanals abschließt.
G50. Das Laborgerät (1) nach einer der vorstehenden Ausführungsformen, umfassend mindestens ein thermisches Isolationsbauteil.
G51. Das Laborgerät (1) nach der vorstehenden Ausführungsform, wobei das mindestens eine Isolationsbauteil zumindest teilweise aus einem thermischen Isolationsmaterial besteht.
G52. Das Laborgerät (1) nach der vorstehenden Ausführungsform und mit den Merkmalen der Ausführungsform G23, wobei das mindestens eine thermische Isolationsbauteil in einem Zwischenraum zwischen dem äußeren Gehäuse (10) und dem inneren Gehäuse (20) angeordnet ist.
G53. Das Laborgerät (1) nach der vorstehenden Ausführungsform mit den Merkmalen der Ausführungsformen G13, und G24, wobei das mindestens eine thermische Isolationsbauteil zwischen den Seitenwänden (22) und dem Seitengehäuse (12), zwischen der Rückwand (28) und dem Rückgehäuse (18), zwischen der unteren Wand (26) und dem Untergehäuse (16), zwischen der Deckenwand (24) und dem Deckengehäuse (14) und/oder zwischen dem Türabschnitt (29) und dem Türgehäuse (19) angeordnet ist.
G54. Das Laborgerät (1) nach einer der vorstehenden Ausführungsformen mit den Merkmalen der Ausführungsform G51, wobei das mindestens eine thermische Isolationsbauteil eine Abschlussschicht aufweist, welche das mindestens eine thermische Isolationsbauteil versiegelt.
G55. Das Laborgerät (1) nach der vorstehenden Ausführungsform, wobei die Abschlussschicht eine Folie umfasst.
G56. Das Laborgerät (1) nach vorstehenden Ausführungsform, wobei die Folie eine Temperaturbeständigkeit bis mindestens 200°C, vorzugsweise bis mindestens 220°C hat.
G57. Das Laborgerät (1) nach einer der vorstehenden Ausführungsformen mit den Merkmalen der Ausführungsformen G5, wobei die Folie flexibel und/oder emissionsarm ausgebildet ist.
G58. Das Laborgerät (1) nach einer der vorstehenden Ausführungsformen mit den Merkmalen der Ausführungsform G55, wobei die Folie eine Klebeschicht umfasst, welche ausgebildet ist, überlappende Lagen der Folie zu verkleben, um das thermische Isolationsbauteil zu versiegeln, insbesondere partikeldicht oder partikelemissionsreduzierend zu versiegeln.
G59. Das Laborgerät (1) nach der vorstehenden Ausführungsform und mit den Merkmalen der Ausführungsformen G51, wobei die Klebeschicht ausgebildet ist, die Abschlussschicht mit dem thermischen Isolationsmaterial zu verkleben.
G60. Das Laborgerät (1) nach einer der vorstehenden Ausführungsformen mit den Merkmalen der Ausführungsform G49, wobei das thermische Isolationsmaterial in die Folie eingeschlagen ist und/oder wobei Überlappungsbereiche der Folie mit einem weiteren Folienabschnitt überdeckend verklebt sind.
G61. Das Laborgerät (1) nach einer der vorstehenden Ausführungsformen mit den Merkmalen der Ausführungsform G55, wobei die Folie eine Kunststofffolie ist, welche vorzugsweise ein Polymer enthält.
G62. Das Laborgerät (1) nach der vorstehenden Ausführungsform, wobei die Folie Polyamid umfasst.
G63. Das Laborgerät (1) nach einer der vorstehenden Ausführungsformen mit den Merkmalen der Ausführungsform G61, wobei die Abschlussschicht mittels eines Polyamidbands verklebt ist.
G64. Das Laborgerät (1) nach einer der vorstehenden Ausführungsformen mit den Merkmalen der Ausführungsform G58, wobei die Klebeschicht eine Silikonklebstoffschicht ist.
G65. Das Laborgerät (1) nach einer der vorstehenden Ausführungsformen mit den Merkmalen der Ausführungsform G3, umfassend eine Steuereinheit, welche ausgebildet ist, eine Förderleistung der Vorrichtung zur Erzeugung der Druckdifferenz an einen Betriebsmodus des Laborgeräts (1) anzupassen.
G66. Das Laborgerät (1) nach der vorstehenden Ausführungsform, wobei die Steuereinheit einen ersten Betriebsmodus aufweist und die Vorrichtung zur Erzeugung des Differenzdrucks ein Gebläse (30) und eine Pumpe (32) umfasst, wobei in dem ersten Betriebsmodus ausschließlich das Gebläse (30) aktiv ist.
G67. Das Laborgerät (1) nach der vorstehenden Ausführungsform, wobei die Steuereinheit einen zweiten Betriebsmodus aufweist, wobei in dem zweiten Betriebsmodus sowohl das Gebläse (30) als auch die Pumpe (32) aktiv sind.
G68. Das Laborgerät (1) nach der vorstehenden Ausführungsform, wobei die Steuereinheit ausgebildet ist bei Erreichen eines Temperaturgrenzwerts von dem ersten Betriebsmodus in den zweiten Betriebsmodus zu wechseln.
G69. Das Laborgerät (1) nach einer der vorstehenden Ausführungsformen mit den Merkmalen der Ausführungsform G4 und G66, wobei eine Förderleistung des Gebläses (30) konstant ist und eine Förderleistung der Pumpe (32) anpassbar ist, um eine Gesamtförderleistung der Vorrichtung zur Erzeugung der Druckdifferenz zu erhöhen.
G70. Das Laborgerät (1) nach einer der vorstehenden Ausführungsformen mit den Merkmalen der Ausführungsform G4 und G66, wobei die Steuereinheit ausgebildet ist, die Pumpe (32) zusätzlich zu dem Gebläse (30) zu schalten, wenn eine vorbestimmte Temperatur des Laborgeräts (1) erreicht wird, um eine Förderleistung zu erhöhen.
G71. Das Laborgerät (1) nach einer der vorstehenden Ausführungsformen mit den Merkmalen der Ausführungsform G3 und G36, wobei die Vorrichtung zur Erzeugung der Druckdifferenz ausgebildet ist, Partikel aus einem Innenraum des äußeren Gehäuses (10), insbesondere dem Türbereich, abzusaugen.
G72. Das Laborgerät (1) nach einer der vorstehenden Ausführungsformen mit den Merkmalen der Ausführungsform G3, wobei die Vorrichtung zur Druckerzeugung eine minimale Förderleistung aufweist, um eine gerichtete Luftströmung von der Umgebung des Laborgeräts (1) in das äußere Gehäuse (10) zu generieren.
G73. Das Laborgerät (1) nach einer der vorstehenden Ausführungsformen mit den Merkmalen der Ausführungsform G33, wobei der Schaltkasten ein Schaltkastenbauteil umfasst und wobei die Vorrichtung zur Druckerzeugung ausgebildet ist, einen Luftstrom in dem Schaltkasten zu erzeugen und den Luftstrom in Abhängigkeit einer Bauteiltemperatur des Schaltkastenbauteils zu steuern.
G74. Das Laborgerät (1) nach der vorstehenden Ausführungsform G67, wobei das Schaltkastenbauteil ein Kühlelement, insbesondere ein Kühlwinkel, ist.
G75. Das Laborgerät (1) nach der vorstehenden Ausführungsform und mit den Merkmalen der Ausführungsform G24, wobei das Kühlelement zumindest teilweise an dem inneren Gehäuse (20) angeordnet ist.
G76. Das Laborgerät (1) nach einer der vorstehenden Ausführungsformen mit den Merkmalen der Ausführungsform G13, umfassend eine Schließvorrichtung (35), welche ausgebildet ist, das Türgehäuse (19) mit einem weiteren Teil des äußeren Gehäuses (10) verschließbar zu verbinden.
G77. Das Laborgerät (1) nach der vorstehenden Ausführungsform, wobei das Laborgerät ausgebildet ist, in der Schließvorrichtung (35) einen Unterdruck zu erzeugen.
G78. Das Laborgerät (1) nach einer der vorstehenden Ausführungsformen, wobei das äußere Gehäuse (10) zumindest teilweise luftdicht ausgebildet ist.
G79. Das Laborgerät (1) nach einer der vorstehenden Ausführungsformen mit den Merkmalen der Ausführungsform G8, wobei der Filter einen Abscheidegrad von mindestens 99%, vorzugsweise von mindestens 99,9%, weiter vorzugsweise von mindestens 99,95%, noch weiter vorzugsweise von mindestens 99,995%, bezogen auf Partikel mit einer Partikelgröße, die am schwersten abzuscheiden sind.
G80. Das Laborgerät (1) nach einer der vorstehenden Ausführungsformen mit den Merkmalen der Ausführungsform G8, wobei der Filter aus einem Fasermaterial ist, beispielsweise aus Glasfaser.

Nachfolgend sind Verwendungsausführungsformen genannt. Diese Ausführungsformen werden mit dem Buchstaben "V", gefolgt von einer Zahl, abgekürzt. Wann immer hier auf "Verwendungsausführungen" Bezug genommen wird, sind diese Ausführungsformen gemeint.
V1. Verwendung des Laborgerätes (1) nach einer der vorstehenden Ausführungsformen in einem Reinraum.
V2. Verwendung des Laborgerätes (1) nach einer der vorstehenden Geräteausführungsformen zur Inkubation von Organismen, Zellen, Bakterien und/oder Viren.
V3. Verwendung des Laborgerätes (1) nach einer der vorstehenden Geräteausführungsformen zur Sterilisation eines inneren Gehäuses des Laborgerätes.

### Kurze Beschreibung der Figuren

Die vorliegende Erfindung wird nun unter Bezugnahme auf die beigefügten Zeichnungen beschrieben, die Ausführungsformen der Erfindung veranschaulichen. Diese Ausführungsformen stellen die vorliegende Erfindung beispielhaft dar und schränken diese nicht ein.
- Fig. 1: zeigt eine schematische vertikale Querschnittsansicht einer Ausführungsform des Laborgeräts;
- Fig. 2: zeigt eine schematische vertikale Querschnittsansicht einer Ausführungsform des Laborgeräts;
- Fig. 3: zeigt eine perspektivische Rückansicht des Laborgeräts;
- Fig. 4: zeigt einen vergrößerten Abschnitt der Fig. 3, in dem insbesondere ein Filter zu sehen ist;
- Fig. 5: zeigt eine perspektivische Vorderansicht des Laborgerätes mit verschlossener Tür;
- Fig. 6: zeigt die perspektivische Vorderansicht mit geöffneter Tür; und
- Fig. 7: zeigt eine schematische horizontale Querschnittsansicht des Laborgerätes.

### Detaillierte Beschreibung der Figuren

Es wird darauf hingewiesen, dass nicht alle Zeichnungen alle Bezugszeichen tragen. Stattdessen sind in einigen der Zeichnungen einige der Bezugszeichen der Kürze und Einfachheit der Darstellung halber weggelassen worden. Ausführungsformen der vorliegenden Erfindung werden nachfolgend unter Bezugnahme auf die beigefügten Zeichnungen beschrieben.

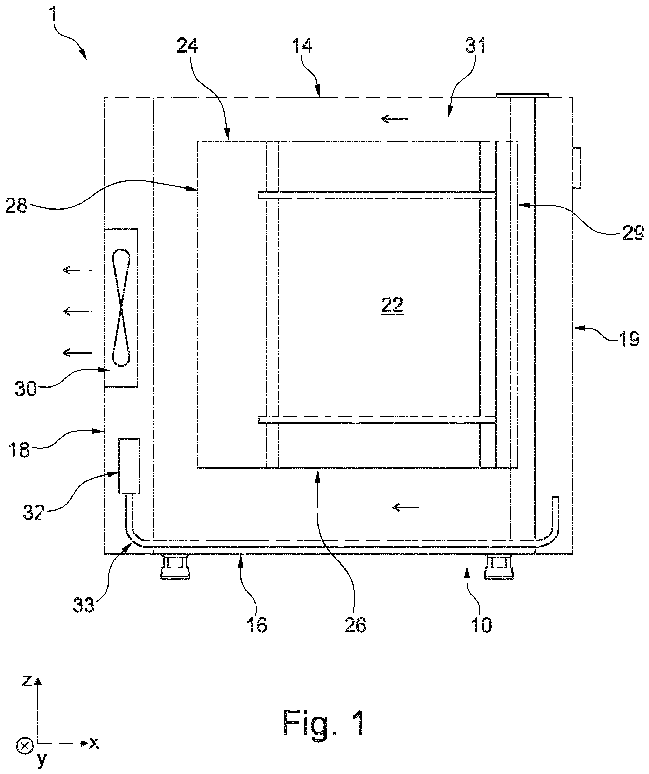

Fig. 1 ist eine schematische Darstellung einer Ausführungsform des Laborgeräts 1 gemäß einer Ausführungsform der Erfindung. Das Laborgerät 1 umfasst ein äußeres Gehäuse 10 welches ein Seitengehäuse 12, ein Rückgehäuse 18, ein Deckengehäuse 14, ein Untergehäuse 16 und ein Türgehäuse 19 aufweist. Das Rückgehäuse 18 und das Türgehäuse 19 sind an entgegengesetzten Enden des Laborgerätes 1 sind. Das Laborgerät 1 umfasst eine Vorrichtung zur Erzeugung der Druckdifferenz, welche ein Gebläse 30 aufweist. Das Laborgerät 1 umfasst weiter ein inneres Gehäuse 20, das Seitenwände 22, eine Rückwand 28, eine untere Wand 26, eine Deckenwand 24 und einen Türabschnitt 29 aufweist. Das innere Gehäuse 20 kann so insbesondere eine innere Kammer, beispielsweise eine Inkubationskammer, definieren.

Zwischen dem äußeren Gehäuse 10 und dem inneren Gehäuse 20 ist ein Zwischenbereich definiert, der einen Rückbereich (zwischen Rückgehäuse 18 und Rückwand), Seitenbereiche (zwischen Seitengehäuse 12 und Seitenwänden 22) und einen Türbereich zwischen dem Türgehäuse 19 und dem Türabschnitt 29 umfasst.

Das Gebläse 30 ist in dem Rückbereich angeordnet, sodass Luft aus dem äußeren Gehäuse in die Laboratmosphäre befördert wird, um innerhalb des äußeren Gehäuse (und insbesondere innerhalb des Zwischenbereichs) einen Unterdruck zu erzeugen. Die Vorrichtung zur Erzeugung der Druckdifferenz, insbesondere das Gebläse 30, erzeugt eine Strömung in Richtung der Vorrichtung zur Erzeugung der Druckdifferenz, insbesondere in Richtung des Rückbereich. Diese Strömung ist insbesondere auch in dem Abschnitt zwischen Untergehäuse 16 und unterer Wand 26, der als Unterbereich bezeichnet wird, und in dem Abschnitt zwischen Deckengehäuse 14 und Deckenwand 24, der als Deckenbereich bezeichnet wird, vorhanden.

Die erzeugte Luftströmung kann Partikel, welche in dem Gerät-Innenraum gelöst werden, in Richtung der Vorrichtung zur Erzeugung der Druckdifferenz transportieren. Der Türbereich kann fluidtechnisch mit dem Rückbereich, dem Unterbereich, dem Deckenbereich und/oder dem Seitenbereich verbunden sein. Die Verbindung kann auch in einem geöffneten Zustand des Laborgeräts 1 bestehen. Ein thermisches Isolationsbauteil kann in dem Gerät-Innenraum 31 angeordnet sein und insbesondere den Zwischenraum zwischen dem äußeren Gehäuse und dem inneren Gehäuse ausfüllen. Hierbei ist eine unvollständige Volumenfüllung vorteilhaft, sodass Luftkanäle zwischen den Flächen der Gehäuse und dem thermischen Isolationsbauteil gebildet sind, welche strömungstechnisch an die Vorrichtung zur Erzeugung des Differenzdrucks gekoppelt sind. Hierzu sind insbesondere Öffnungen zwischen den verschiedenen Bereichen des äußeren Gehäuses vorgesehen. Der von der Vorrichtung zur Erzeugung des Differenzdrucks in Richtung der Laboratmosphäre gerichtete Luftstrom kann durch einen Filter geleitet werden.

Das innere Gehäuse 20 kann ein Gestell zur Aufnahme von Probenhaltern, insbesondere zur Aufnahme von Tabletteinschüben, welche vorzugsweise aus Metall bestehen, aufweisen.

Der Rückbereich kann einen Schaltkasten bilden, welcher durch eine weitere Wandung von dem Gerät-Innenraum abgetrennt ist. Eine Luftströmung von dem Gerät-Innenraum in den Schaltkasten kann durch Öffnungen in der weiteren Wandung realisiert sein. Die Vorrichtung zur Erzeugung der Druckdifferenz ist zumindest in Form des Gebläses in dem Schaltkasten angeordnet.

Die Vorrichtung zur Erzeugung der Druckdifferenz kann eine Pumpe 32 umfassen, die insbesondere als Unterdruckpumpe bzw. Vakuumpumpe ausgeführt sein kann. Die Pumpe 32 kann in dem Schaltschrank angeordnet sein und ist strömungstechnisch mit dem Gebläse gekoppelt. Diese Kopplung kann beispielsweise durch einen Pumpenauslass, welcher in den Schaltschrank gerichtet ist, realisiert sein, sodass Abluft von der Pumpe 32 mittels des Gebläses durch den Filter transportiert werden kann.

Die Pumpe 32 kann eingangsseitig mit einem Schlauch 33 verbunden sein, welcher zumindest teilweise in dem Gerät-Innenraum 31 angeordnet ist. Der Schlauch kann in den Türbereich geführt sein, um in dem Türbereich einen Unterdruck zu erzeugen und/oder einen Luftstrom zum Absaugen von Partikeln aus dem Türbereich zu erzeugen oder zu verstärken. Insbesondere während einer Heizphase oder Sterilisationsphase eines Inkubators kann der Türbereich eine erhöhte Partikelemission aufweisen, wobei die Partikel durch den Schlauch 33 abtransportiert werden können. An der Verbindung von Untergehäuse 16 und Türgehäuse 19 kann ein Verbindungsabschnitt vorgesehen sein, welcher die entsprechenden Bereiche strömungstechnisch verbindet. In dem Verbindungsteil kann ferner der Schlauch 33 angeordnet sein. Ferner kann der Verbindungsabschnitt sichelförmig ausgebildet sein und ein Innenraum des Verbindungsabschnitts kann gegen das Türgehäuse 19 und/oder das Untergehäuse 16 gedichtet sein, um mittels des Verbindungsabschnitts eine Strömungsverbindung zwischen dem Türgehäuse 19 und dem Untergehäuse 16 herzustellen.

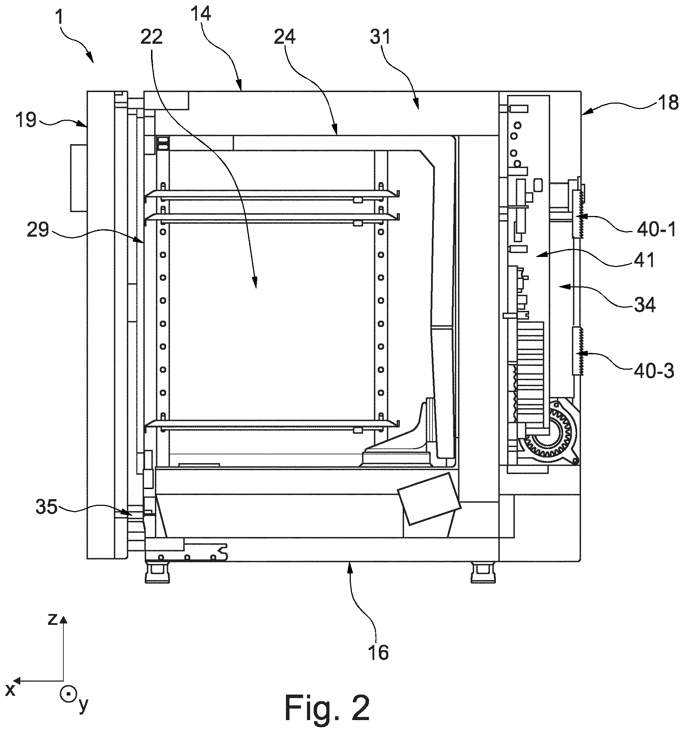

Fig. 2 zeigt eine weitere schematische Querschnittsansicht des Laborgeräts 1, wobei die Tür in dieser Ansicht links angeordnet ist - dementsprechend ist diese Ansicht bezüglich der Ansicht der Fig. 1 um 180° um die z-Achse gedreht. In dem Rückgehäuse 18 des Laborgeräts 1 ist ein Gehäuseabteil 34 vorgesehen, welches einen permanenten Abluftauslass aufweist. Der Filter kann an dem Abluftauslass angeordnet sein. Ferner ist an dem Türgehäuse 19 eine luftgefüllte Schließvorrichtung 35 angeordnet. Mit der Schließvorrichtung 35 kann das Türgehäuse an dem Untergehäuse 16 mechanisch verriegelt werden, um ein unbeabsichtigtes Öffnen des Laborgeräts 1 zu unterbinden. Ferner kann die Schließvorrichtung abgeschlossen werden, um einen unbefugten Zugriff auf das Innere des Laborgeräts zu unterbinden. Mit einem Schließen der Schließvorrichtung 35 kann die Dichtung 29 mit einem statischen Druck beaufschlagt werden. Das Untergehäuse 16 kann in Form eines Doppelbodens ausgebildet sein, wobei eine äußere Wandung gegen die Laboratmosphäre gedichtet ist. Ein durch den Doppelboden gebildetes Volumen kann strömungstechnisch mit Rückbereich, respektive dem Schaltkasten gekoppelt sein. Eine thermische Isolierung in Form von thermischen Isolationsbauteilen kann in dem Gerät-Innenraum 31 angeordnet sein. Insbesondere kann - wie bereits beschrieben - der Gerät-Innenraum fluidisch gekoppelt sein, sodass gas von einem Bereich des Geräte-Innenraums in einen anderen Bereich des Geräte-Innenraums befördert werden kann. Mit einer strömungstechnischen Kopplung kann ein Luftstrom mittels der Vorrichtung zur Erzeugung des Differenzdrucks, welche in dem Schaltkasten angeordnet ist, in dem Gerät-Innenraum erzeugt werden. Mit einer fluidtechnischen Kopplung kann zumindest ein homogener Druck, insbesondere ein Unterdruck, innerhalb des Gerät-Innenraums erreicht werden. Die Tür kann eine Dichtung aufweisen, welche die Tür gegen das Seitengehäuse 12 abdichtet. Die Seitenwand 22 kann Aufnahmen für Tabletteinschübe aufweisen.

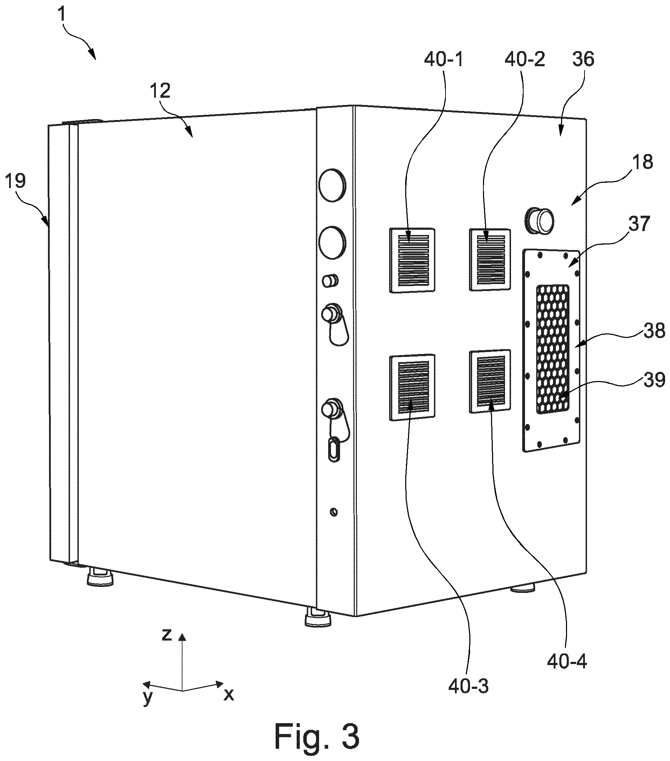

Fig. 3 zeigt eine perspektivische Rückansicht einer Ausführungsform des Laborgeräts 1. Insbesondere ist an der Rückseite 36 des Laborgeräts 1 ein Filterhalter 37 angeordnet, welcher an dem Rückgehäuse 18 mittels einer lösbaren Verbindung, insbesondere einer Mehrzahl von Schraubverbindungen, befestigt ist. Der Filterhalter 37 umfasst einen Rahmen 38, auf welchem die die Schraubverbindungen angeordnet sind. Ferner umfasst der Filterhalter eine Filteröffnung, an welcher ein Schutzgitter 39 angeordnet ist. Der Filterhalter 37 kann auf einer Gehäusefläche des Rückgehäuses 18 aufsetzen. Der Filter 50 und/oder das Schutzgitter 39 können von einer Oberfläche des Filterhalters 37 nach innen versetzt sein. Das Schutzgitter 39 kann eine periodische, insbesondere hexagonales Gitterstegstruktur aufweisen.

Weiterhin sind auf der Gehäusefläche des Rückgehäuses 18 Lüftungsöffnungen 40-1, 40-2, 40-3, 40-4 vorgesehen, durch welche Luft in das Laborgerät, insbesondere in das Rückgehäuse 18, respektive den Schaltschrank strömen kann. Diese Luftströmung kann verwendet werden, um elektronische Bauteile innerhalb des Schaltschranks zu kühlen. Laborluft, welche über die Lüftungsöffnungen 40-1, 40-2, 40-3, 40-4 angesaugt wird, kann über den Filter 50 gefiltert an die Laboratmosphäre abgegeben werden.

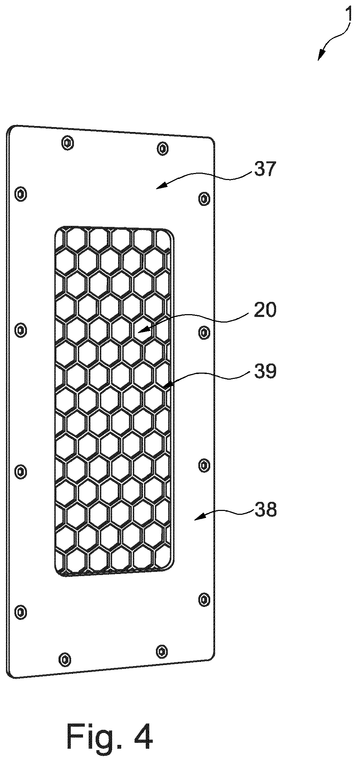

Fig. 4 zeigt einen Ausschnitt einer Ausführungsform des Rückgehäuses 18. Der Rahmen 38 des Filterhalters 37 ist mit einer Mehrzahl von Haltemitteln, insbesondere Schraub- oder Nietverbindungen, an dem Rückgehäuse 18 befestigt. Der Filterhalter 37 ist lösbar ausgestaltet, um den Filter wechseln zu können. Die Haltemittel können umlaufend an einer Kante des Filterhalters 37 angeordnet sein. Die Haltemittel können in Bezug auf einen Umfang des Filterhalters 37 im Wesentlichen äquidistant angeordnet sein.

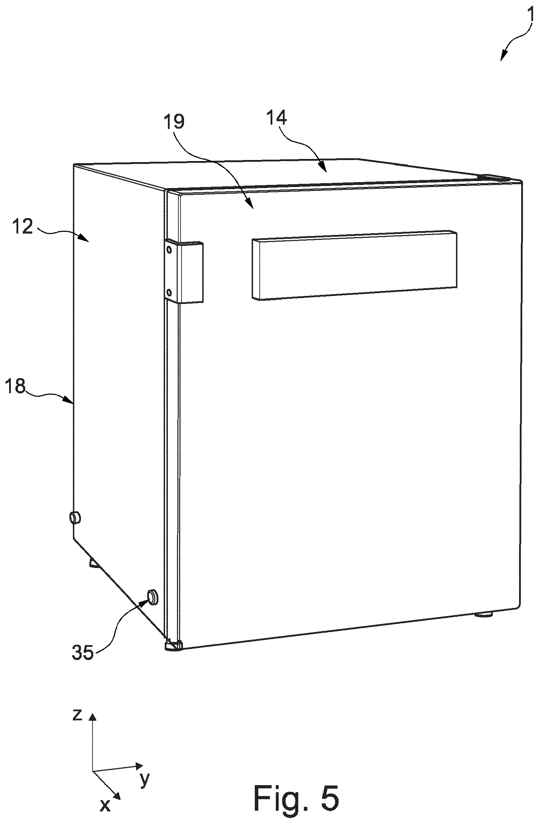

Fig. 5 zeigt eine perspektivische Frontansicht einer Ausführungsform des Laborgeräts 1. Das Türgehäuse 19 umfasst ein Benutzerinterface mit einer Anzeige und Eingabemitteln. Die Schließvorrichtung 35 kann einen Schließzylinder aufweisen, welcher eine an dem Seitengehäuse 12 angeordnete Schlüsselöffnung aufweist. Die Schließvorrichtung 35 kann über die Schlüsselöffnung abschließbar sein, sodass das Türgehäuse 19 in einem geschlossenen Zustand nicht geöffnet werden kann.

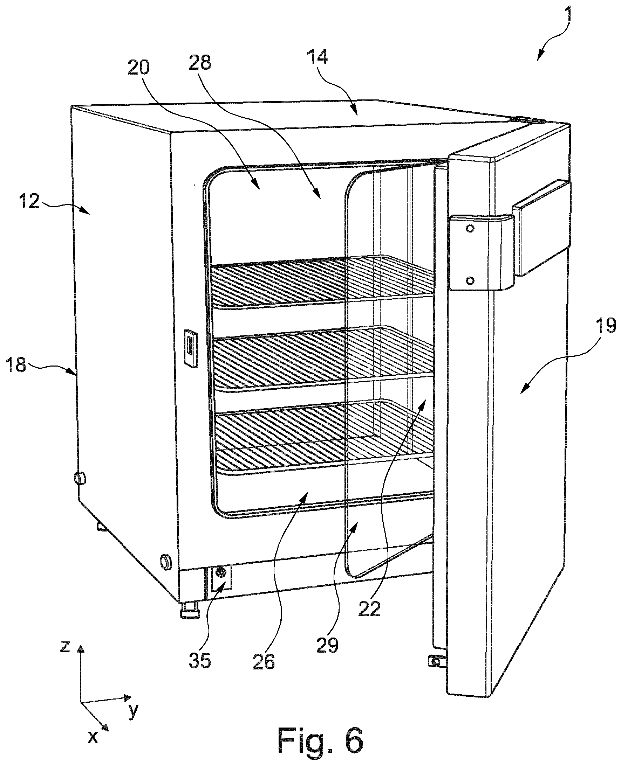

Fig. 6 zeigt eine perspektivische Frontansicht einer Ausführungsform des Laborgeräts 1. Der Türabschnitt 29 umfasst eine Glastür, welche zum Schließen des inneren Gehäuses 20 geeignet ist. Die Glastür kann auf einer vorderen Gerätefläche, welche das Deckengehäuse 14, das Untergehäuse 16 und/oder das Seitengehäuse 12 abschließt zur Anlage kommen. An der vorderen Gerätefläche kann eine Dichtung angeordnet sein, an welcher die Glastür in einem geschlossenen Zustand zur Anlage kommen kann, sodass die Glastür das innere Gehäuse 20 dichtend abschließt.

Die Schließvorrichtung 35 kann eine Schließöffnung in dem Unterbereich aufweisen, in welche ein Stift der Schließvorrichtung, welcher an der Tür angeordnet ist, eingreifen kann. Mit geschlossener Tür kann der in der Schließöffnung angeordnete Stift verriegelt werden. Das Unterabschnitt kann ferner einen separat angesetztes Gehäuseteil aufweisen, in welchem auch die Schließöffnung angeordnet sein kann. Das Seitengehäuse 12 kann das äußere Gehäuse 10 in einer vollen Gerätehöhe seitlich abschließen, wobei ein Untergehäuse 16 zwischen den gegenüberliegenden Seitengehäusen 12 angeordnet sein kann.

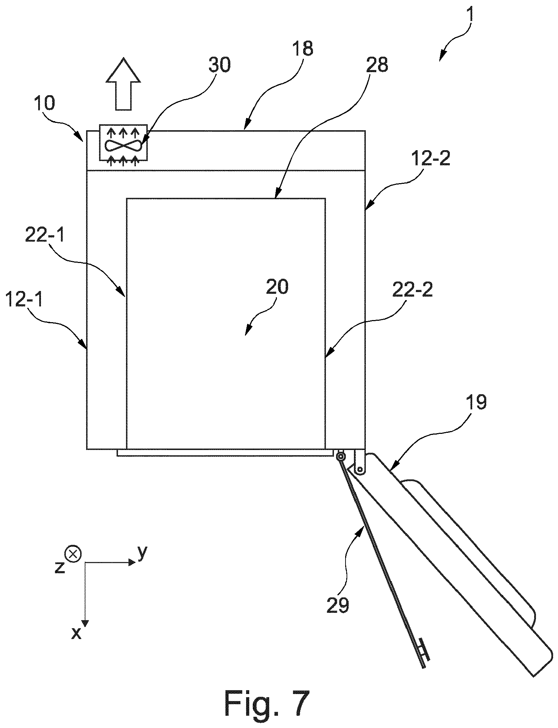

Fig. 7 zeigt eine schematische horizontale Querschnittsansicht einer Ausführungsform des Laborgeräts 1 von oben. Die Tür ist mittels einer Aufhängevorrichtung an dem Seitengehäuse 12 schwenkbar befestigt, um das äußere Gehäuse 10 zu öffnen und Zugang zu dem inneren Gehäuse 20 zu ermöglichen. Der Zwischenbereich zwischen dem inneren Gehäuse 20 und dem äußeren Gehäuse 10

Die Bereiche im Zwischenraum zwischen dem äußeren Gehäuse und dem inneren Gehäuse können zumindest teilweise mit Isolationsbauteilen, insbesondere mit thermischem Isoliermaterial, versehen sein. Dementsprechend ist das Gebläse 30 strömungstechnisch mit dem Zwischenberiech verbunden, um einen Partikeltransport zu einem Filter zu leiten, welcher in Bezug auf den Luftstrom stromabwärts von dem Gebläse 30 angeordnet sein kann. Wie bereits beschrieben können Teilbereiche (beispielsweise innerhalb der Tür) auch über Schläuche mit einer Pumpe verbunden werden, sodass über die Schläuche Partikel zu der Pumpe transportiert werden können, und ein Auslass der Pumpe kann strömungstechnisch an das Gebläse gekoppelt sein.

Wenn in diesem Dokument ein relativer Begriff wie "etwa", "im Wesentlichen" oder "ungefähr" verwendet wird, soll dieser Begriff auch den genauen Begriff einschließen. Das heißt, z. B. "im Wesentlichen gerade" sollte so ausgelegt werden, dass er auch "(genau) gerade" einschließt.

Während im Vorstehenden eine bevorzugte Ausführungsform unter Bezugnahme auf die Zeichnungen beschrieben wurde, wird der Fachmann verstehen, dass diese Ausführungsform nur zur Veranschaulichung bereitgestellt wurde und keinesfalls so ausgelegt werden sollte, dass sie den Bereich der vorliegenden Erfindung, der durch die Ansprüche definiert ist, einschränkt.

## Patentansprüche

1. Laborgerät (1), wobei das Laborgerät (1) ein Inkubator (1) ist, wobei das Laborgerät (1) ein äußeres Gehäuse (10) aufweist, das einen Gerät-Innenraum definiert, wobei das Laborgerät (1) ausgelegt ist, einen Betriebszustand einzunehmen, bei dem ein Druck in dem Gerät-Innenraum geringer ist als ein Umgebungsdruck in der Umgebung des Laborgerätes (1).

2. Das Laborgerät (1) nach Anspruch 1, wobei das Laborgerät (1) eine Vorrichtung zur Erzeugung der Druckdifferenz aufweist, wobei die Vorrichtung zur Erzeugung der Druckdifferenz ein Gebläse (30) und/oder eine Pumpe (32) umfasst.

3. Das Laborgerät (1) nach Anspruch 2, wobei die Vorrichtung zur Erzeugung der Druckdifferenz dazu ausgelegt ist, Gas aus dem Gerät-Innenraum in die Umgebung des Laborgeräts (1) zu fördern, wobei das Laborgerät (1) einen Filter (50) aufweist, der zwischen einem Auslass der Vorrichtung zur Erzeugung der Druckdifferenz und der Umgebung des Laborgerätes (1) angeordnet ist.

4. Das Laborgerät (1) nach einem der vorstehenden Ansprüche, wobei das Laborgerät ein Gesamtvolumen im Bereich von 0,1 m³ bis 2,5 m³, vorzugsweise im Bereich von 0,2 m³ bis 1,0 m³ und weiter vorzugsweise im Bereich von 0,4 m³ bis 0,8 m³ hat.

5. Das Laborgerät (1) nach einem der vorstehenden Ansprüche, wobei das Laborgerät (1) ein inneres Gehäuse (20) aufweist, welches eine Kammer definiert, wobei in dem Betriebszustand der Druck im Geräte-Innenraum in einem Bereich vorhanden ist, der außen durch das äußere Gehäuse (10) und innen durch das innere Gehäuse (20) begrenzt wird, und wobei dieser Druck geringer ist als ein Druck in der Kammer.

6. Das Laborgerät (1) nach Anspruch 5, wobei das äußere Gehäuse (10)
ein Seitengehäuse (12),
ein Rückgehäuse (18),
ein Deckengehäuse (14),
ein Untergehäuse (16) und
ein Türgehäuse (19) aufweist,
wobei das Rückgehäuse (18) und das Türgehäuse (19) an entgegengesetzten Enden des Laborgerätes (1) angeordnet sind,
wobei das innere Gehäuse (20)
Seitenwände (22),
eine Rückwand (28),
eine untere Wand (26),
eine Deckenwand (24) und
einen Türabschnitt (29) aufweist,
wobei das Laborgerät (1) ausgebildet ist, einen Unterdruck in einem Türbereich zu erzeugen, der durch das Türgehäuse (19) und den Türabschnitt (29) begrenzt wird.

7. Das Laborgerät (1) nach einem der vorstehenden Ansprüche mit den Merkmalen des Anspruchs 2, wobei das Laborgerät (1) mindestens einen Schlauch umfasst, der die Vorrichtung zur Erzeugung des Differenzdruckes fluidisch mit mindestens einem anderen Bereich verbindet.

8. Das Laborgerät (1) nach Anspruch 7 im Rückbezug zum Anspruch 6, wobei der mindestens eine Schlauch den Türbereich und die Vorrichtung zur Erzeugung des Differenzdrucks fluidisch verbindet.

9. Das Laborgerät nach einem der Ansprüche 7 und 8, wobei der mindestens eine Schlauch aus einem Material ist, das eine Temperaturbeständigkeit bis mindestens 200° C, vorzugsweise bis mindestens 220 ° C hat.

10. Das Laborgerät (1) nach einem der vorstehenden Ansprüche, umfassend mindestens ein thermisches Isolationsbauteil, wobei das mindestens eine thermische Isolationsbauteil eine Abschlussschicht aufweist, welche das mindestens eine thermische Isolationsbauteil versiegelt, wobei die Abschlussschicht eine Folie umfasst, wobei die Folie eine Temperaturbeständigkeit bis mindestens 200°C, vorzugsweise bis mindestens 220°C hat.

11. Das Laborgerät (1) nach einem der vorstehenden Ansprüche mit den Merkmalen des Anspruchs 2, umfassend eine Steuereinheit, welche ausgebildet ist, eine Förderleistung der Vorrichtung zur Erzeugung der Druckdifferenz an einen Betriebsmodus des Laborgeräts (1) anzupassen, wobei die Steuereinheit einen ersten Betriebsmodus aufweist und die Vorrichtung zur Erzeugung des Differenzdrucks ein Gebläse (30) und eine Pumpe (32) umfasst, wobei in dem ersten Betriebsmodus ausschließlich das Gebläse (30) aktiv ist, wobei die Steuereinheit einen zweiten Betriebsmodus aufweist, wobei in dem zweiten Betriebsmodus sowohl das Gebläse (30) als auch die Pumpe (32) aktiv sind, wobei die Steuereinheit ausgebildet ist bei Erreichen eines Temperaturgrenzwerts von dem ersten Betriebsmodus in den zweiten Betriebsmodus zu wechseln.

12. Verwendung des Laborgerätes (1) nach einem der vorstehenden Ansprüche in einem Reinraum.
